# EUROPEAN PATENT SPECIFICATION

(11) **EP 1 262 478 B1**
(45) Date of publication and mention of the grant of the patent: **12.11.2008**
(21) Application number: 02010958.3
(22) Date of filing: 26.09.1997
(51) Int. Cl.: C12Q 1/00, G01N 33/50

(54) **Methods and compositions for determining cellular response profiles**
Verfahren und Zusammensetzungen zur Bestemmung von zellülaren Response-Profile
Procedes et compositions pour determiner les profils de reaction cellulaire

(30) Priority: 26.09.1996 US 719697
(43) Date of publication of application: 04.12.2002
(62) Divisional of application: 97943625.0
(73) Proprietor: Vertex Pharmaceuticals (San Diego) LLC, San Diego, California 92121 (US)
(72) Inventor: Whitney, Michael A., San Diego, CA 92117 (US); Negulescu, Paul A., Del Mar, CA 92014 (US); Craig, Frank, Solana Beach, CA 92075 (US); Mere, Lora, San Diego, CA 92122 (US); Foulkes, Gorden J., Encinitas, CA 92024 (US)
(74) Representative: Schnappauf, Georg

(56) References cited:
- WO-A-97/06277
- US-A- 5 487 992
- REDDY ET AL: "Fluorescence activated sorting of totipotent embryonic stem cells expressing developmentaly regulated lacZ fusion genes" PROCEEDINGS OF THE NATIONAL ACADEMY OF SCIENCES OF USA, NATIONAL ACADEMY OF SCIENCE. WASHINGTON, US, vol. 89, August 1992 (1992-08), pages 6721-6725, XP002115386 ISSN: 0027-8424
- SKARNES W C ET AL: "A GENE TRAP APPROACH IN MOUSE EMBRYONIC STEM CELLS: THE LACZ REPORTER IS ACTIVATED BY SPLICING REFLECTS ENDOGENOUS GENE EXPRESSION AND IS MUTAGENIC IN MICE" GENES AND DEVELOPMENT, COLD SPRING HARBOR, NY, US, vol. 6, no. 6, 1 June 1992 (1992-06-01), pages 903-918, XP000569059 ISSN: 0890-9369

## Description

### Technical Field

The present application generally relates to methods and compositions for the identification of useful and functional portions of the genome and compounds for modulating such portions of the genome, particularly the identification of proteins that are directly or indirectly transcriptionally regulated and compounds for regulating such proteins, either directly or indirectly.

### Background

The identification and isolation of useful portions of the genome requires extensive expenditure of time and financial resources. Currently, many genome projects use various strategies to reduce cloning and sequencing times. While genome projects rapidly expand the database of genetic material, such projects often lack the ability to integrate the information with the biology of the cell or organism from which the genes were isolated. In some instances, coding regions of newly isolated genes reveal sequence homology to other genes of known function. This type of analysis can, at best, provide clues as to the possible relationships between different genes and proteins. Genomic projects in general, however, suffer from the inability to rapidly and directly isolate, and identify specific, yet unknown, genes associated with particular a biological process or processes.

The evaluation of the function of genes identified from genomic sequencing projects requires cloning the discovered gene into an expression system suitable for functional screening. Transferring the discovered gene into a functional screening system requires additional expenditure of time and resources without a guarantee that the correct screening system was chosen. Since the function of the discovered gene is often unknown or only surmised by inference to structurally related genes, the chosen screening system may not have any relationship to the biological function of the gene. For example a gene may encode a protein that is structurally homologous to the beta-adrenergic receptor and have a dissimilar function. Further, if negative results are obtained in the screen, it can not be easily determined whether 1) the gene or gene product is not functioning properly in the screening assay or 2) the gene or gene product is directly or indirectly involved in the biological process being assayed by the screening system.

Consequently, there is a need to provide methods and compositions for rapidly isolating portions of genomes associated with a known biological process and to screen such portions of genomes for activity without the necessity of transferring the gene of interest into an additional screening system.

### Brief Description of the Figures

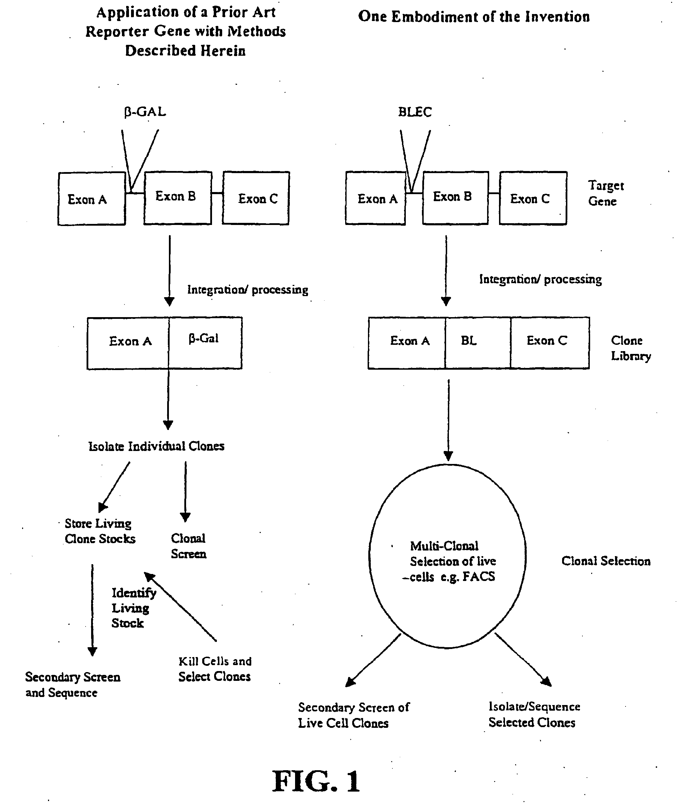

**FIG. 1** shows a comparison between an application of a prior art reporter gene with methods described herein, and one embodiment of the invention. The prior art uses the b-gal reporter and requires the establishment of clones prior to expression analysis. One embodiment of this invention allows for the rapid identification of living cell clones from large multiclonal populations of **BLEC** (beta-lactamase expression construct) integrated cells. This is a significant advancement over the prior art, which requires the analysis of individual clones followed by the retrieving of selected clone from a duplicate clonal stock of living cells.

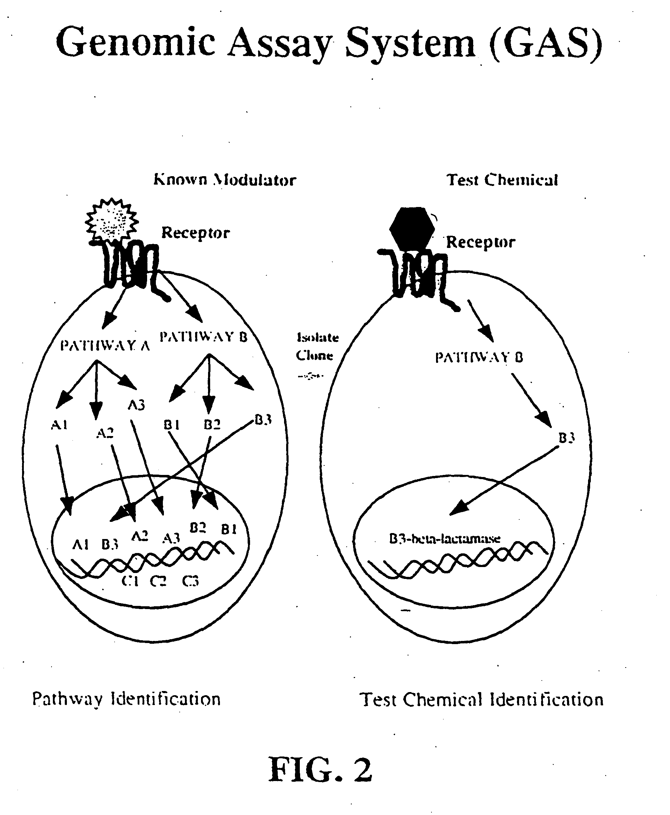

**FIG. 2** shows a representation of how one embodiment of the invention reports the expression of a pathway within a cell and can be used for screening.

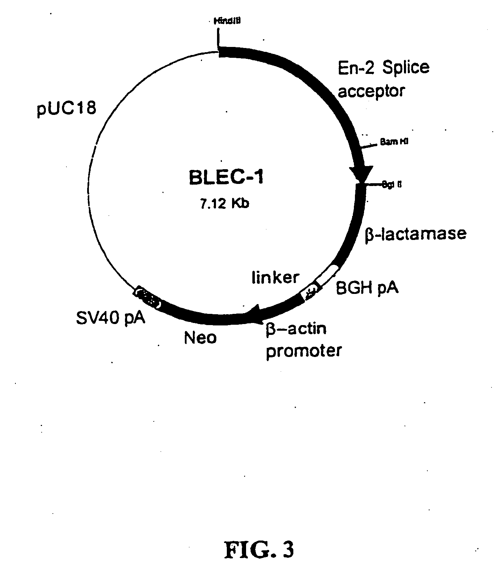

**FIG. 3** shows a schematic plasmid map of the **BLEC-1.**

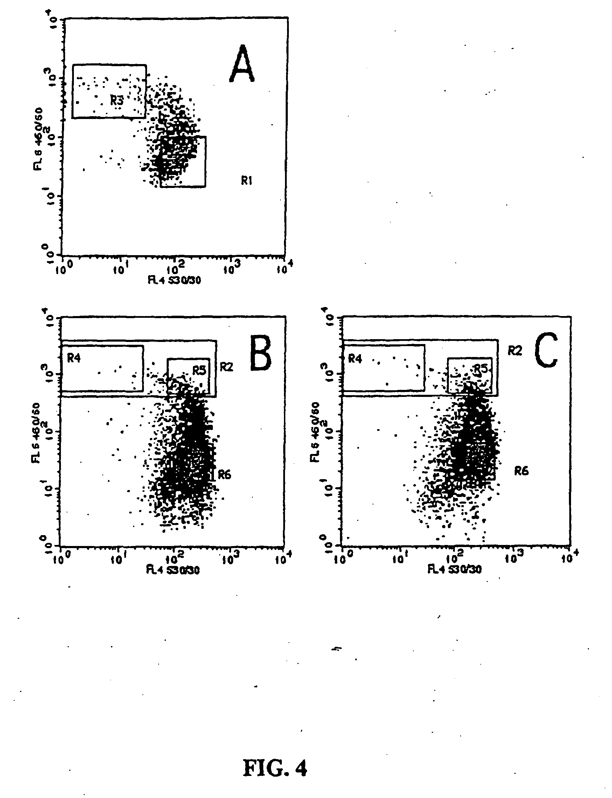

**FIG. 4** shows the FACS analysis of a population of genomically **BLEC** integrated clones. Individually cells are plotted by fluorescent emission properties at 400 nm excitation. The x axis represents green emission (530 nm). The y axis represents blue emission (465 nm). Cells with a high blue/green ration will appear blue in color and cells with a low blue/green ratio will appear green in color. **A)** Unselected multiclonal population of **BLEC** integrated RBL-1 cell clones. **B)** Population of clones sorted from **3A (R1)** that were cultured for an additional 7 days and resorted. **C)** Population from **3B** with addition of 1uM ionomycin for 12 hours prior to sorting.

### Summary

The present application recognizes that β-lactamase polynucleotides can be effectively used in living eukaryotic cells to functionally identify active portions of a genome directly or indirectly associated with a biological process. The present application also recognizes for the first time that β-lactamase activity can be measured using membrane permeant substrates in living cells incubated with a test chemical that directly or indirectly interacts with a portion of the genome having an integrated β-lactamase polynucleotide. The present application, thus, permits the rapid identification and isolation of genomic polynucleotides indirectly or directly associated with a defined biological process and identification of compounds that modulate such processes and regions of the genome. Methods and compositions for estimating the physiological specificity of candidate drugs have also been described in WO 97/06277. Because the identification of active genomic polynucleotides is permitted in living cells, further functional characterization can be conducted using the same cells, and optionally, the same screening assay. The ability to functionally screen immediately after the rapid identification of a functionally active portion of a genome, without the necessity of transferring the identified portion of the genome into a secondary screening system, represents, among other things, a distinct advantage over an application of a prior art reporter gene with the methods described herein, as shown in **FIG. 1****.**

The application provides for a method of identifying portions of a genome, e.g. genomic polynucleotides, in a living cell using a polynucleotide encoding a protein with β-lactamase activity that can be detected with a membrane permeant β-lactamase substrate. Typically, the method involves inserting a polynucleotide encoding a protein with β-lactamase activity into the genome of an organism using any method known in the art, developed in the future or described herein. Usually, a β-lactamase expression construct will be used into integrate a β-lactamase polynucleotide into a eukaryotic genome, as described herein. The cell, such as a eukaryotic cell, is usually contacted with a predetermined concentration of a modulator, either before or after integration of the β-lactamase polynucleotide. β-lactamase activity is usually then measured inside the living cell, preferably with fluorescent, membrane permeant β-lactamase substrates that are transformed by the cell into membrane impermeant β-lactamase substrates as described herein.

The application also provides for a method of identifying proteins or compounds that directly or indirectly modulate a genomic polynucleotide. Generally, the method comprises inserting a β-lactamase expression construct into an eukaryotic genome, usually non-yeast, contained in at least one living cell, contacting the cell with a predetermined concentration of a modulator, and detecting β-lactamase activity in the cell.

The application also provides for a method of screening compounds with an active genomic polynucleotide that comprises: 1) optionally contacting a multiclonal population of cells with a first test chemical prior to separating said cells by a FACS, 2) separating by a FACS said multiclonal population of cells into β-lactamase expressing cells and non β-lactamase expressing cells, wherein said β-lactamase expressing cells have a detectable difference in cellular fluorescence properties compared to non-β-lactamase expressing cells, 3) contacting either population of cells with the same or a different test chemical, and 4) optionally repeating step (2), wherein said multi-clonal population of cells comprises eukaryotic cells having a β-lactamase expression construct integrated into a genome of said eukaryotic cells and a membrane permanent β-lactamase substrate transformed inside said cells to a membrane impermeant β-lactamase substrate. The steps of this method can be repeated to permit additional characterization of identified clones.

The application also includes powerful methods and compositions for identifying physiologically relevant cellular pathways and proteins of interest of known, unknown or partially known function. As shown in **FIG. 2** a pathway may have more than one major intracellular signal. Two major intracellular pathways are shown ("A" and "B"). Each intracellular signal pathway may also have multiple branches. Each arm is shown as having three signaling pathways (A1, A2, and A3; and B1, B2, and B3). By generating a library of clones with a -lactamase expression construct, genomic polynucleotides for each signal pathway can be tagged or reported by the expression of -lactamase. Pathways not effected by the modulator (shown as C1, C2, and C3) are also tagged with -lactamase expression construct. Because the modulator only modulates the expression of pathways A1, A2, A3, B1, B2, and B3, only clones corresponding to these genomic integration sites are identified as being responsive to the modulator. Clones corresponding to sites C1, C2, and C3 remain unaltered and are not responsive to the modulator. Any individual, modulated clone can be immediately isolated, if not already isolated, and used for a drug discovery assay to screen test chemicals for activity for modulating the reported pathway, as described herein.

The application also includes tools for pathway identification and drug discovery that can be applied to a number of targets of interest and therapeutic areas including, proteins of interest, physiological responses even in the absence of a definitive target (e.g. immune response, signal transduction, neuronal function and endocrine function), viral targets, and orphan proteins.

### Detailed Description of the Invention

### Definitions

Unless defined otherwise, all technical and scientific terms used herein have the same meaning as commonly understood by one of ordinary skill in the art to which this invention belongs. Generally, the nomenclature used herein and the laboratory procedures in cell culture, molecular genetics, and nucleic acid chemistry and hybridization described below are those well known and commonly employed in the art. Standard techniques are used for recombinant nucleic acid methods, polynucleotide synthesis, and microbial culture and transformation (e.g., electroporation, lipofection). Generally, enzymatic reactions and purification steps are performed according to the manufacturer's specifications. The techniques and procedures are generally performed according to conventional methods in the art and various general references (see generally, Sambrook et al. Molecular Cloning: A Laboratory Manual, 2d ed. (1989) Cold Spring Harbor Laboratory Press, Cold Spring Harbor, N.Y., ) which are provided throughout this document. The nomenclature used herein and the laboratory procedures in analytical chemistry, organic synthetic chemistry, and pharmaceutical formulation described below are those well known and commonly employed in the art. Standard techniques are used for chemical syntheses, chemical analyses, pharmaceutical formulation and delivery, and treatment of patients. As employed throughout the disclosure, the following terms, unless otherwise indicated, shall be understood to have the following meanings:
"Fluorescent donor moiety" refers to a fluorogenic compound or part of a compound (including a radical) which can absorb energy and is capable of transferring the energy to another fluorogenic molecule or part of a compound. Suitable donor fluorogenic molecules include, but are not limited to, coumarins and related dyes xanthene dyes such as fluoresceins, rhodols, and rhodamines, resorufins, cyanine dyes, bimanes, acridines, isoindoles, dansyl dyes, aminophthalic hydrazides such as luminol and isoluminol derivatives, aminophthalimides, aminonaphthalimides, aminobenzofurans, aminoquinolines, dicyanohydroquinones, and europium and terbium complexes and related compounds.
"Quencher" refers to a chromophoric molecule or part of a compound that is capable of reducing the emission from a fluorescent donor when attached to the donor. Quenching may occur by any of several mechanisms including fluorescence resonance energy transfer, photoinduced electron transfer, paramagnetic enhancement of intersystem crossing, Dexter exchange coupling, and exciton coupling such as the formation of dark complexes.
"Acceptor" refers to a quencher that operates via fluorescence resonance energy transfer. Many acceptors can re-emit the transferred energy as fluorescence. Examples include coumarins and related fluorophores, xanthenes such as fluoresceins, rhodols, and rhodamines, resorufins, cyanines, difluoroboradiazaindacenes, and phthalocyanines. Other chemical classes of acceptors generally do not re-emit the transferred energy. Examples include indigos, benzoquinones, anthraquinones, azo compounds, nitro compounds, indoanilines, di- and triphenylmethanes.
"Dye" refers to a molecule or part of a compound that absorbs specific frequencies of light, including but not limited to ultraviolet light. The terms "dye" and "chromophore" are synonymous.
"Fluorophore" refers to a chromophore that fluoresces.
"Membrane-permeant derivative" refers a chemical derivative of a compound of that increases membrane permeability of the compound. These derivatives are made better able to cross cell membranes, i.e. membrane permeant, because hydrophilic groups are masked to provide more hydrophobic derivatives. Also, the masking groups are designed to be cleaved from the fluorogenic substrate within the cell to generate the derived substrate intracellularly. Because the substrate is more hydrophilic than the membrane permeant derivative it is now trapped within the cells.
"Isolated polynucleotide" refers to a polynucleotide of genomic, cDNA, or synthetic origin or some combination there of, which by virtue of its origin the "isolated polynucleotide" (1) is not associated with the cell in which the "isolated polynucleotide" is found in nature, or (2) is operably linked to a polynucleotide which it is not linked to in nature.
"Isolated protein" refers to a protein of cDNA, recombinant RNA, or synthetic origin or some combination thereof, which by virtue of its origin the "isolated protein" (1) is not associated with proteins found it is normally found with in nature, or (2) is isolated from the cell in which it normally occurs or (3) is isolated free of other proteins from the same cellular source, e.g. free of human proteins, or (4) is expressed by a cell from a different species, or (5) does not occur in nature.
"Polypeptide" as used herein as a generic term to refer to native protein, fragments, or analogs of a polypeptide sequence. Hence, native protein, fragments, and analogs are species of the polypeptide genus. Preferred, β-lactamase polypeptides include those with the polypeptide sequence represented in the **SEQUENCE ID**. **LISTING** and any other polypeptide or protein having similar β-lactamase activity as measured by one or more of the assays described herein. β-lactamase polypeptide or proteins can include any protein having sufficient activity for detection in the assays described herein.
"Naturally-occurring" as used herein, as applied to an object, refers to the fact that an object can be found in nature. For example, a polypeptide or polynucleotide sequence that is present in an organism (including viruses) that can be isolated from a source in nature and which has not been intentionally modified by man in the laboratory is naturally-occurring.
"Operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences.
"Control sequence" refers to polynucleotide sequences which are necessary to effect the expression of coding and non-coding sequences to which they are ligated. The nature of such control sequences differs depending upon the host organism; in prokaryotes, such control sequences generally include promoter, ribosomal binding site, and transcription termination sequence; in eukaryotes, generally, such control sequences include promoters and transcription termination sequence. The term "control sequences" is intended to include, at a minimum, components whose presence can influence expression, and can also include additional components whose presence is advantageous, for example, leader sequences and fusion partner sequences.
"Polynucleotide" refers to a polymeric form of nucleotides of at least 10 bases in length, either ribonucleotides or deoxynucleotides or a modified form of either type of nucleotide. The term includes single and double stranded forms of DNA. "Genomic polynucleotide" refers to a portion of a genome. "Active genomic polynucleotide" or "active portion of a genome" refer to regions of a genome that can be up regulated, downregulated or both, either directly or indirectly, by a biological process. "Directly," in the context of a biological process or processes, refers to direct causation of a process that does not require intermediate steps, usually caused by one molecule contacting or binding to another molecule (the same type or different type of molecule). For example, molecule A contacts molecule B, which causes molecule B to exert effect X that is part of a biological process. "Indirectly," in the context of a biological process or processes, refers to indirect causation that requires intermediate steps, usually caused by two or more direct steps. For example, molecule A contacts molecule B to exert effect X which in turn causes effect Y.
"β-lactamase polynucleotide" refers to a polynucleotide encoding a protein with β-lactamase activity. Preferably, the protein with β-lactamase activity can measured be in a FACS at 22°degrees Celsius using a CCF2-AM β-lactamase substrate at a level of about 1,000 such protein molecules or less per cell. More preferably, the protein with β-lactamase activity can measured be in a FACS at 22° degrees Celsius using a CCF2-AM β-lactamase substrate at a level of about 300 to 1,000 such protein molecules per cell. More preferably, the protein with β-lactamase activity can measured be in a FACS at 22° degrees Celsius using a CCF2-AM β-lactamasesubstrate at a level of about 25 to 300 such protein molecules per cell. Proteins with β-lactamaseactivity that require more than 1,000 molecules of such protein per cell for detection with a FACS at 22° degrees Celsius using a CCF2-AM β-lactamase substrate can be used and preferably have at least 5% of the activity of the protein with **SEQ. ID. NO.: 1.**
"Sequence homology" refers to the proportion of base matches between two nucleic acid sequences or the proportion amino acid matches between two amino acid sequences. When sequence homology is expressed as a percentage, e.g., 50%, the percentage denotes the proportion of matches over the length of sequence from a desired sequence (e.g. β-lactamase sequences, such as **SEQ. ID. NO.: 1**) that is compared to some other sequence. Gaps (in either of the two sequences) are permitted to maximize matching; gap lengths of 15 bases or less are usually used, 6 bases or less are preferred with 2 bases or less more preferred. When using oligonucleotides as probes or treatments the sequence homology between the target nucleic acid and the oligonucleotide sequence is generally not less than 17 target base matches out of 20 possible oligonucleotide base pair matches (85%); preferably not less than 9 matches out of 10 possible base pair matches (90%), and most preferably not less than 19 matches out of 20 possible base pair matches (95%).
"Selectively hybridize" refers to detectably and specifically bind. Polynucleotides, oligonucleotides and fragments thereof selectively hybridize to target nucleic acid strands, under hybridization and wash conditions that minimize appreciable amounts of detectable binding to nonspecific nucleic acids. High stringency conditions can be used to achieve selective hybridization conditions as known in the art and discussed herein. Generally, the nucleic acid sequence homology between the polynucleotides, oligonucleotides, and fragments thereof and a nucleic acid sequence of interest will be at least 30%, and more typically with preferably increasing homologies of at least about 40%, 50%, 60%, 70%, and 90%.

Typically, hybridization and washing conditions are performed at high stringency according to conventional hybridization procedures. Positive clones are isolated and sequenced. For illustration and not for limitation, a full-length polynucleotide corresponding to the nucleic acid sequence of **SEQ. ID.NO. 1** may be labeled and used as a hybridization probe to isolate genomic clones from a the appropriate target library in λEMBL4 or λGEM11 (Promega Corporation, Madison, Wisconsin); typical hybridization conditions for screening plaque lifts (Benton and Davis (1978) Science 196: 180) can be: 50% formamide, 5 x SSC or SSPE, 1-5 x Denhardt's solution, 0.1-1% SDS, 100-200 µg sheared heterologous DNA or tRNA, 0-10% dextran sulfate, 1 x10⁵ to 1 x 10⁷ cpm/ml of denatured probe with a specific activity of about 1 x 10⁸ cpm/µg, and incubation at 42°C for about 6-36 hours. Prehybridization conditions are essentially identical except that probe is not included and incubation time is typically reduced. Washing conditions are typically 1-3 x SSC, 0.1-1% SDS, 50-70°C with change of wash solution at about 5-30 minutes. Cognate sequences, including allelic sequences, can be obtained in this manner.

Two amino acid sequences are homologous if there is a partial or complete identity between their sequences. For example, 85% homology means that 85% of the amino acids are identical when the two sequences are aligned for maximum matching. Gaps (in either of the two sequences being matched) are allowed in maximizing matching; gap lengths of 5 or less are preferred with 2 or less being more preferred. Alternatively and preferably, two protein sequences (or polypeptide sequences derived from them of at least 30 amino acids in length) are homologous, as this term is used herein, if they have an alignment score of at more than 5 (in standard deviation units) using the program ALIGN with the mutation data matrix and a gap penalty of 6 or greater. See Dayhoff, M.O., in Atlas of Protein Sequence and Structure, 1972, volume 5, National Biomedical Research Foundation, pp. 101-110, and Supplement 2 to this volume, pp. 1-10. The two sequences or parts thereof are more preferably homologous if their amino acids are greater than or equal to 30% identical when optimally aligned using the ALIGN program.

"Corresponds to" refers to a polynucleotide sequence is homologous (i.e., is identical, not strictly evolutionarily related) to all or a portion of a reference polynucleotide sequence, or that a polypeptide sequence is identical to all or a portion of a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

The following terms are used to describe the sequence relationships between two or more polynucleotides: "reference sequence," "comparison window," "sequence identity," "percentage of sequence identity," and "substantial identity." A "reference sequence" is a defined sequence used as a basis for a sequence comparison; a reference sequence may be a subset of a larger sequence, for example, as a segment of a full-length cDNA or gene sequence given in a sequence listing such as a **SEQ. ID. NO.: 1**, or may comprise a complete cDNA or gene sequence. Generally, a reference sequence is at least 20 nucleotides in length, frequently at least 25 nucleotides in length, and often at least 50 nucleotides in length. Since two polynucleotides may each (1) comprise a sequence (i.e., a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window", as used herein, refers to a conceptual segment of at least 20 contiguous nucleotide positions wherein a polynucleotide sequence may be compared to a reference sequence of at least 20 contiguous nucleotides and wherein the portion of the polynucleotide sequence in the comparison window may comprise additions or deletions (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman (1981) Adv. Appl. Math. 2: 482, by the homology alignment algorithm of Needleman and Wunsch (1970) J. Mol. Biol. 48: 443, by the search for similarity method of Pearson and Lipman (1988) Proc. Natl. Acad. Sci. (U.S.A.) 85: 2444, by computerized implementations of these algorithms (GAP, BESTFIT, FASTA, and TFASTA in the Wisconsin Genetics Software Package Release 7.0, Genetics Computer Group, 575 Science Dr., Madison, WI), or by inspection, and the best alignment (i.e., resulting in the highest percentage of homology over the comparison window) generated by the various methods is selected. The term "sequence identity" means that two polynucleotide sequences are identical (i.e., on a nucleotide-by-nucleotide basis) over the window of comparison. The term "percentage of sequence identity" is calculated by comparing two optimally aligned sequences over the window of comparison, determining the number of positions at which the identical nucleic acid base (e.g., A, T, C, G, U, or I) occurs in both sequences to yield the number of matched positions, dividing the number of matched positions by the total number of positions in the window of comparison (i.e., the window size), and multiplying the result by 100 to yield the percentage of sequence identity. The terms "substantial identity" as used herein denotes a characteristic of a polynucleotide sequence, wherein the polynucleotide comprises a sequence that has at least 30 percent sequence identity, preferably at least 50 to 60 percent sequence identity, more usually at least 60 percent sequence identity as compared to a reference sequence over a comparison window of at least 20 nucleotide positions, frequently over a window of at least 25-50 nucleotides, wherein the percentage of sequence identity is calculated by comparing the reference sequence to the polynucleotide sequence which may include deletions or additions which total 20 percent or less of the reference sequence over the window of comparison.

As applied to polypeptides, the term "substantial identity" means that two peptide sequences, when optimally aligned, such as by the programs GAP or BESTFIT using default gap weights, share at least 30 percent sequence identity, preferably at least 40 percent sequence identity, more preferably at least 50 percent sequence identity, and most preferably at least 60 percent sequence identity. Preferably, residue positions, which are not identical, differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, glutamic-aspartic, and asparagine-glutamine.

"Polypeptide fragment" refers to a polypeptide that has an amino-terminal and/or carboxy-terminal deletion, but where the remaining amino acid sequence is usually identical to the corresponding positions in the naturally-occurring sequence deduced, for example, from a full-length cDNA sequence (e.g., the sequence shown in **SEQ. ID. NO.**: 1). "β-lactamase polypeptides fragment" refers to a polypeptide that is comprised of a segment of at least 25 amino acids that has substantial identity to a portion of the deduced amino acid sequence shown in **SEQ. ID. NO.:1** and which has at least one of the following properties: (1) specific binding to a β-lactamase substrate, preferably cephalosporin, under suitable binding conditions, or (2) the ability to effectuate enzymatic activity, preferably cephalosporin backbone cleavage activity, when expressed in a mammalian cell. Typically, analog polypeptides comprise a conservative amino acid substitution (or addition or deletion) with respect to the naturally occurring sequence. Analogs typically are at least 300 amino acids long, preferably at least 500 amino acids long or longer, most usually being as long as full-length naturally-occurring polypeptide.

"Modulation " refers to the capacity to either enhance or inhibit a functional property of a biological activity or process (e.g., enzyme activity or receptor binding). Such enhancement or inhibition may be contingent on the occurrence of a specific event, such as activation of a signal transduction pathway, and/or may be manifest only in particular cell types.

The term "modulator" refers to a chemical (naturally occurring or non-naturally occurring), such as a biological macromolecule (e.g. nucleic acid, protein, non-peptide, or organic molecule), or an extract made from biological materials such as bacteria, plants, fungi, or animal (particularly mammalian) cells or tissues. Modulators are typically evaluated for potential activity as inhibitors or activators (directly or indirectly) of a biological process or processes (e.g., agonist, partial antagonist, partial agonist, antagonist, antineoplastic agents, cytotoxic agents, inhibitors of neoplastic transformation or cell proliferation, cell proliferation-promoting agents, and the like) by inclusion in assays described herein. The activity of a modulator may be known, unknown or partial known.

The term "test chemical" refers to a chemical to be tested by one or more method(s) of the invention as a putative modulator. A test chemical is usually not known to bind to the target of interest. The term "control test chemical" refers to a chemical known to bind to the target (e.g., a known agonist, antagonist, partial agonist or inverse agonist). Such control chemicals include chemicals that 1) non-specifically or substantially disrupt protein structure (e.g., denaturing agents (e.g., urea or guandium), charotropic agents, sulfhydryl reagents (e.g., dithiotritol and β-mercaptoethanol), and proteases), 2) generally inhibit cell metabolism (e.g., mitochondrial uncouplers) and 3) non-specifically disrupt electrostatic or hydrophobic interactions of a protein (e.g., high salt concentrations, or detergents at concentrations sufficient to non-specifically disrupt hydrophobic interactions). Preferably, a "test chemical" is not a chemical known to be unsuitable for a therapeutic use for a particular indication due to toxicity of the subject. Usually, various predetermined concentrations of test chemicals are used for screening such as .01 µM, .1 µM, 1.0 µM, and 10.0 µM.

The term "target" refers to a biochemical entity involved in a biological process. Preferably, targets are proteins that play a useful role in the physiology or biology of an organism. A therapeutic chemical binds to target to alter or modulate its function. As used herein, targets can include cell surface receptors, G-proteins, kinases, ion channels, phopholipases and other proteins mentioned herein.

The terms "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or enzymatic activity that can be detected by optical or colorimetric methods). Various methods of labeling polypeptides and glycoproteins are known in the art and may be used. Examples of labels for polypeptides include, but are not limited to, the following: radioisotopes (e.g., ³H, ¹⁴C, ³⁵S, ¹²⁵I, ¹³¹I), fluorescent labels (e.g., FITC, rhodamine, and lanthanide phosphors), enzymatic labels (or reporter genes) (e.g., enzymatic reporter genes horseradish peroxidase, β-galactosidase, luciferase and alkaline phosphatase; and non-enzymatic reporter genes (e.g., fluorescent proteins)), chemiluminescent, biotinyl groups, predetermined polypeptide epitopes recognized by a secondary reporter (e.g., leucine zipper pair sequences, binding sites for secondary antibodies, metal binding domains, epitope tags). "Substantially pure" refers to an object species is the predominant species present (i.e., on a molar basis it is more abundant than any other individual species in the composition), and preferably a substantially purified fraction is a composition wherein the object species comprises at least about 50 percent (on a molar basis) of all macromolecular species present. Generally, a substantially pure composition will comprise more than about 80 percent of all macromolecular species present in the composition, more preferably more than about 85%, 90%, 95%, and 99%. Most preferably, the object species is purified to essential homogeneity (contaminant species cannot be detected in the composition by conventional detection methods) wherein the composition consists essentially of a single macromolecular species.

"Pharmaceutical agent or drug" refers to a chemical or composition capable of inducing a desired therapeutic effect when properly administered (e.g. using the proper amount and delivery modality) to a patient.

Other chemistry terms herein are used according to conventional usage in the art, as exemplified by The McGraw-Hill Dictionary of Chemical Terms (ed. Parker, S., 1985), McGraw-Hill, San Francisco,.

### Introduction

The present application recognizes that β-lactamase polynucleotides can be effectively used in living eukaryotic cells to functionally identify active portions of a genome directly or indirectly associated with a biological process. The present application also recognizes for the first time that β-lactamase activity can be measured using membrane permeant substrates in living cells incubated with a test chemical that directly or indirectly interacts with a portion of the genome having an integrated β-lactamase polynucleotide. The present application, thus, permits the rapid identification and isolation of genomic polynucleotides indirectly or directly associated with a defined biological process and identification of compounds that modulate such processes and regions of the genome. Because the identification of active genomic polynucleotides is permitted in living cells, further functional characterization can be conducted using the same cells, and optionally, the same screening assay. The ability to functionally screen immediately after the rapid identification of a functionally active portion of a genome, without the necessity of transferring the identified portion of the genome into a secondary screening system, represents, among other things, a distinct advantage over an application of a prior art reporter gene and methods described herein, as shown in **FIG. 1**.

The application includes several general and useful aspects, including:
1) a method for identifying genes or gene products directly or indirectly associated (e.g. regulated) with a biological process of interest (that can be modulated by a compound) using a genomic polynucleotide operably linked to a polynucleotide encoding a protein with β-lacatamase activity,
2) a method for identifying proteins (e.g. orphan proteins or known proteins) or compounds that directly or indirectly modulate (e.g. activate or inhibit transcription) a genomic polynucleotide operably linked to a polynucleotide encoding a protein with β-lactamase activity,
3) a method of screening for an active genomic polynucleotide (e.g. enhancer, promoter or coding region in the genome) that can be directly or indirectly associated (e.g. regulated) with a biological process of interest (that can be modulated by a compound) using a genomic polynucleotide operably linked to a polynucleotide encoding a protein with β-lactamase activity that can be detected by FACS using a fluorescent, membrane permeant β-lactamase substrate,
4) eukaryotic cells with a genomic polynucleotide operably linked to a polynucleotide encoding a protein with β-lactamase activity, and
5) polynucleotides related to the above methods and cells.

These aspects as well as others described herein, can be achieved by using the methods and compositions of matter described herein. will be further recognized that various aspects can be combined to make desirable embodiments, for example, a method of identifying compounds that modulate active genomic polynucleotides operably linked to a protein with β-lactamase activity that can be detected by FACS using a fluorescent, membrane permeant β-lactamase substrate.

### Methods for Rapidly Identifying Functional Portions of a Genome

A method of identifying portions of a genome, e.g. genomic polynucleotides, in a living cell using a polynucleotide encoding a protein with β-lactamase activity that can be detected with a membrane permeant β-lactamase substrate is provided. Typically, the method involves inserting a polynucleotide encoding a protein with β-lactamase activity into the genome of an organism using any method known in the art, developed in the future or described herein. Usually, a β-lactamase expression construct will be used into integrate a β-lactamase polynucleotide into a eukaryotic genome, as described herein. The cell, such as a eukaryotic cell, is usually contacted with a predetermined concentration of a modulator, either before or after integration of the β-lactamase polynucleotide. β-lactamase activity is usually then measured inside the living cell, preferably with fluorescent, membrane permeant β-lactamase substrates that are transformed by the cell into membrane impermeant β-lactamase substrates as described herein and PCT Publication No. WO96/30540 published October 3, 1996, by Tsien et al.

Once β-lactamase polynucleotides are integrated into the genome of interest, they become under the transciptional control of the genome of the host cell. Integration into the genome is usually stable, as described herein and known in the art. Transcriptional control of the genome often results from receptor (e.g. intracellular or cell surface receptor) activation, which can regulate transcriptional and translational events to change the amount of protein present in the cell. The amount of protein present with β-lactamase activity can be measured via its enzymatic action on a substrate. Normally, the substrate is a small uncharged molecule that, when added to the extracellular solution, can penetrate the plasma membrane to encounter the enzyme. A charged molecule can also be employed, but the charges are generally masked by groups that will be cleaved by endogenous or heterologous cellular enzymes or processes (e.g., esters cleaved by cytoplasmic esterases). As described more fully herein and in PCT Publication No. WO96/30540 published October 3, 1996, by Tsien et al., the use of substrates that exhibit changes in their fluorescence spectra upon interaction with an enzyme are particularly desirable. In some assays, the fluorogenic substrate is converted to a fluorescent product by β-lactamase activity. Alternatively, the fluorescent substrate changes fluorescence properties upon conversion by β-lactamase activity. Preferably, the product should be very fluorescent to obtain a maximal signal, and very polar, to stay trapped inside the cell.

### Vectors and Integration

Vectors, such as viral and plasmid vectors, can be used to introduce genes or genetic material of the invention into cells, preferably by integration into the host cell genome. Such viral vectors can be any appropriate viruses, such as retroviruses, adenoviruses, adeno-associated viruses, papillomaviruses, herpes viruses, or any ecotropic or amphitropic virus, preferably a retrovirus. The viruses can be, for example, retroviruses or any other virus modified to be replicatively deficient, cytomegalovirus, Friend leukemia virus, SIV, HIV, Rouse Sarcoma Virus, or Maloney virus such as Moloney murine leukemia virus.

Vectors, such as retrovirus vectors, can encode an operable selective protein so that cells that have been transformed can be positively selected for. Such selective protein can be antibiotic resistance factors, such as neomycin resistance, such as *NEO*. Alternatively, cells can be negatively selected for using an enzyme, such as herpes simplex virus thymidine kinase (HSVTK) that transforms a pro-toxin into a toxin. Viral vectors, such as retroviral vectors, are available that are suitable for these purposes, such as PSIR vector (available from ClonTech of California with PT67 packaging cells) GgU3Hisen and GgTNKneoU3 and GgTKNeoen variants of Moloney murine leukemia virus, are available. Vector modifications can be made that allow more efficient integration into the host cell genome. Such modifications include sequences that enhance integration or known methods to promote nucleic acid transportation into the nucleus of the host cell. Retro-viral vectors are those described in U.S. Patent Number 5,364,783 by Ruley and von Melchner can also be used to increase transfection efficiency.

Vectors can also be used with liposomes or other vesicles that can transport genetic material into a cell. Appropriate structures are known in the art. The liposomes can include vectors such as plasmids or yeast artificial chromosomes (YACs), which can include genetic material to be introduced into the cell. Plasmids can also be introduced into cells by any known methods, such as electroporation, calcium phosphate, or lipofection. DNA fragments, without a plasmid or viral vector can also be used.

In one aspect of the present invention, vectors are used to introduce reporter genes into cells. When the reporter gene integrates into the genome of a target cell so that the reporter gene is expressed, that event can be detected by detecting the reporter gene. Clones that express the reporter gene under a wide variety of conditions can be used for a variety of purposes, including gene and drug discovery. Chromosomes tagged with β-lactamase expression constructs can be transferred to desired receipt cells using methods established in the art.

β-lactamase polynucleotides can be placed on a variety of plasmids for integration into a genome and to identify genes from a large variety of organisms (Gorman, C.M. et al., Mol. Cell Biol. 2: 1044-1051 (1982); Alam, J. and Cook, J.L., Anal.Biochem. 188: 245-254, (1990)). Standard techniques are used to introduce these polynucleotides into a cell or whole organism (e.g., as described in Sambrook, J., Fritsch, E.F. and Maniatis, T. Expression of cloned genes in cultured mammalian cells. In: Molecular Cloning, edited by Nolan, C. New York: Cold Spring Harbor Laboratory Press, 1989). Resistance markers can be used to select for successfully transfected cells.

If a β-lactamase expression construct is selected for integrating a β-lactamase polynucleotide into a eukaryotic genome, it will usually contain at least a β-lactamase polynucleotide operably linked to a splice acceptor and optionally a splice donor. Alternatively, the β-lactamase polynucleotide may be operably linked to any means for integrating a polynucleotide into a genome, preferably for integration into an intron of a gene to produce an in frame translation product. The β-lactamase expression construct can optionally comprise, depending on the application, an IRES element, a splice donor, a poly A site, translational start site (e.g. a Kozak sequence) an LTR (long terminal repeat) and a selectable marker.

### β-Lactamase Reporter Genes

Preferably, β-lactamase polynucleotides encode a cytosolic form of a protein with β-lactamase activity. This provides the advantage of trapping the normally secreted β-lactamase protein within the cell, which enhances signal to noise ratio of the signal associated with β-lactamase activity. Usually, this is accomplished by removing or disabling the signal sequence normally present for secretion. As used herein, "cytosolic protein with β-lactamase activity " refers to a protein with β-lactamase activity that lacks the proper amino acid sequences for secretion from the cell, e.g., the signal sequence. For example, in the polypeptide of **SEQ. ID NO.: 1**, the signal sequence has been replaced with the amino acids Met-Ser. Accordingly, upon expression, β-lactamase activity remains within the cell. For expression in mammalian cells it is preferably to use β-lactamase polynucleotides with nucleotide sequences preferred by mammalian cells. In some instances, a secreted form of β-lactamase can be used with the methods and compositions of the invention. In particular, genes having sequences that direct selection can be identified with a (word) β-lactamase assay. This also permits multiplying based on directed localization of β-lactamase.

Proteins with β-lactamase activity can be any known to the art, developed in the future or described herein. This includes, for example, the enzymes represented by **SEQ. ID. NO.'s** described herein. Nucleic acids encoding proteins with β-lactamase activity can be obtained by methods known in the art, for example, by polymerase chain reaction of cDNA using primers based on the DNA sequence in **SEQ. ID. NO.: 1**. PCR methods are described in, for example, U.S. Pat. No. 4,683,195; Mullis et. al. (1987) Cold Spring Harbor Symp. Quant. Biol. 51:263; and Erlich, ed., PCR Technology, (Stockton Press, NY, 1989).

### Sequences for Assisting Integration

The β-lactamase expression construct typically includes sequences for integration, especially sequences designed to target or enhance integration into the genome. The splice site acceptor can be operably linked to the β-lactamase polynucleotide to facilitate expression upon integration into an intron. Usually, a fusion RNA will be created with the coding region of an adjacent operably portion of the exon. A splice acceptor sequence is a sequence at the 3' end of an intron where it junctions with an exon. The consensus sequences for a splice acceptor is NTN (TC) (TC) (TC) TTT (TC) (TC)(TC) (TC) (TC) (TC) NCAGgt. The intronic sequences are represented by upper case and the exonic sequence by lower case font. These sequences represent those that are conserved from viral to primate genomes.

The splice donor site can be operably linked to the β-lactamase polynucleotide to facilitate integration in an intron to promote expression by requiring a poly-adenylation sequence. Usually, a fusion RNA is created with the coding region or untranslated on the 3' end of the β-lactamase polynucleotide. This is preferred when it is desired to sequence the coding region of the identified gene. A splice donor is a sequence at the 5' end of an intron where it junctions with an exon. The consensus sequence for a splice donor sequence is naggt(ag)aGT. The intronic sequences are represented by upper case and the exonic sequence by lower case font. These sequences represent those that are conserved from viral to primate genomes. This splice donor allows identification of the target gene using 3' RACE.

As an alternative to a splice donor site, a poly A site may be operably linked to the β-lactamase polynucleotide. Poly-adenylation signals, i.e poly A sites, include SV40 poly A sites, such as those described in the Invitrogen Catalog 1996 (California). In some instances, it may be desirable to include in the β-lactamase expression construct a translational start site. For instance, a translational start site allows for β-lactamase expression even if the integration occurs in non-coding regions. Usually, such sequences will not reduce the expression of a highly expressed gene. Translational start sites include a "Kozak sequence" and are the preferred sequences for expression in mammalian cells described in Kozak, M., J. Cell Biol. 108: 229-241 (1989). The nucleotide sequence for a cytosolic protein with β-lactamase activity in **SEQ. ID. NO.: 3** contains a Kozak sequences for the nucleotides -9 to 4 (GGTACCACCATGA).

It is also preferable, when using mammalian cells, to include an IRES ("internal ribosome entry binding site") element in the β-lactamase expression construct. Typically, an IRES element will improve the yield of expressing clones. One caveat of integration vectors is that only one in three insertions into an intron will be in frame and produce a functional reporter protein. This limitation can be reduced by cloning an IRES sequence between the splice acceptor site and the reporter gene (e.g., a β-lactamase polynucleotide). This eliminates reading frame restrictions and possible functional inactivation of the reporter protein by fusion to an endogenous protein. IRES elements include those from piconaviruses, picorna-related viruses, and hepatitis A and C. Preferably, the IRES element is from a poliovirus. Specific IRES elements can be found, for instance, in W09611211 by Das and Coward published 4/16/96, EP 585983 by Zurr published 3/7/96, WO9601324 by Berlioz published 1/18/96 and WO9424301 by Smith published October 27, 1994,. To improve selection of β-lactamase polynucleotide into a genome, a selectable marker can be used in the β-lactamase expression construct. Selectable markers for mammalian cells are known in the art, and include for example, thymidine kinase, dihydrofolate reductase (together with methotrexate as a DHFR amplifier), aminoglycoside phosphotransferase, hygromycin B phosphotransferase, asparagine synthetase, adenosine deaminase, metallothionien, and antibiotic resistant genes such as genes neomycin resistance. Selectable markers for non-mammalian cells are known in the art and include genes providing resistance to antibiotics, such as kanamycin, tetracycline, and ampicillin

The application can be readily practiced on genomes having intron/exon structures. Such genomes include those of mammals (e.g., human, rabbit, mouse, rat, monkey, pig and cow), vertebrates, insects and yeast. Intron-targeted vectors are more commonly used in mammalian cells as introns, or intervening sequences, are considerably larger than exons, or mRNA coding regions in mammals. Intron targeting can be achieved by cloning a splice acceptor or 3' intronic sequences upstream of a β-lactamase polynucleotide gene followed by a polyadenylation signal or 5' intronic splice donor site. When the vector inserts into an intron, the reporter gene (e.g., β-lactamase) is expressed under the same control as the gene into which it has inserted.

The application can also be practiced on genomes having reduced numbers of, or lacking, intron/exon structures. For lower eukaryotes, which have simple genomic organization, i.e. containing few and small introns, exon-targeted vectors can be used. Such vectors include β-lactamase polynucleotides operably linked to a poly-adenylation sequence and optionally to an IRES element. Lower eukaryotes include yeast, and fungi and pathogenic eurokaryotes (e.g. parasites and microoganisms). For genomes lacking intron/exon structures restriction enzyme integration, transposon induced integration or selection integration can be used for genomic integration. Such methods include those described by Kuspa and Loomis, PNAS 89: 8803-8807 (1992) and Derbyshire, K.M., Gene Nov. 7: 143-144 (1995). Prokaryotes can be used with the invention if integration can occur in such genomes. Retroviral vectors can also be used to integrate β-lactamase polynucleotides into a genome (e.g., eukaryotic), such as those methods and composition described in U.S. Patent Number 5,364,783.

Typically, integration will occur in the regions of the genome that are accessible to the integration vector. Such regions are usually active portions of the genome where there is increased genome regulatory activity, e.g. increased polymerase activity or a change in DNA binding by proteins that regulate transcription of the genome. Many embodiments of the invention described herein can result in random integration, especially in actively transcribed regions.

### Integration into Active Portions of the Genome

Integration, however, can be directed to regions of the genome active during specific types of genome activity. For instance, integration at sites in the genome that are active during specific phases of the cell cycle can be promoted by synchronizing the cells in a desired phase of the cell cycle. Such cell cycle methods include those known in the art, such as serum deprivation or alpha factors (for yeast). Integration may also be directed to regions of the genome active during cell regulation by a chemical, such as an antagonist or agonist for a receptor or some other chemical that increase or decreases or otherwise modulates genome activity. By adding the chemical of interest, genome activity can be increased, often in specific regions to promote integration of an integration vector (e.g. as a reporter gene construct), into such regions of the genome.

For instance, a nuclear receptor activator (general or specific) could applied to activate the cells prior or during integration in order to promote integration of reporter genes at sites in the genome that become more active during nuclear receptor activation. Such cells could then be screened with the same or different nuclear receptor activator to identify which clones, and which portions of the genome are active during nuclear receptor activation. Any agonists, antagonists and modulators of the receptors described herein can be used in such a manner, as well as any other chemicals that increase or decrease genome activity.

### Cells for Integration into the Genome

The cells used will typically correspond to the genome of interest. For example, if regions of the human genome are desired to be identified, then human cells containing a proper genetic complement will generally be used. Libraries, however, could be biased by using cells that contain extra-copies of certain chromosomes or other portions of the genome. Cells that do not correspond to the genome of interest can also be used if the genome of interest or significant portions of the genome of interest can be replicated in the cells, such as making a human-mouse hybrid.

Additionally, by the appropriate choice of cells and expressed proteins, identification and screening assays can be constructed that detect active portions of the genome associated with a biological process that requires, in whole or part, the presence of a particular protein (protein of interest). Cells can be selected depending on the type of proteins that are expressed (homologously or heterologously) or from the type of tissue from which the cell line or explant was originally generated. If the identification of portions of the genome activated by a particular type of protein is desired, then the cell used should express that protein.

The cells can express the protein homologously, i.e. expression of the desired protein normally or naturally occurs in the cells. Alternatively, the cells can be directed to express a protein heterologously, i.e. expression of the desired protein which does not normally or naturally occur in the cells. Such heterologous expression can be directed by "turning on" the gene in the cell encoding the desired protein or by transfecting the cell with a polynucleotide encoding the desired protein (either by constitutive expression or inducible expression). Inducible expression is preferred if it is thought that the expressed protein of interest may be toxic to the cells.

Many cells can be used. Such cells include, but are not limited to adult, fetal, or non human embryonic cells. These cells can be derived from the mesoderm, ectoderm, or endoderm and can be stem cells, such as non human embryonic or adult stem cells, or adult precursor cells. The cells can be of any lineage, such as vascular, neural, cardiac, fibroblasts, lymphocytes, hepatocytes, cardiac, hematopoeitic, pancreatic, epidermal, myoblasts, or myocytes. Other cells include baby hamster kidney (BHK) cells (ATCC No. CCL10), mouse L cells (ATCC No. CCL1.3), Jurkats (ATCC No. TIB 152) and 153 DG44 cells (see, Chasin (1986) Cell. Molec. Genet. 12: 555) human embryonic kidney (HEK) cells (ATCC No. CRL1573), Chinese hamster ovary (CHO) cells (ATCC Nos. CRL9618, CCL61, CRL9096), PC12 cells (ATCC No. CRL17.21) and COS-7 cells (ATCC No. CRL1651). Preferred cells include Jurkat cells, CHO cells, neuroblastoma cells, P19 cells, F11 cells, NT-2 cells, and HEK 293 cells, such as those described in U.S. Patent No. 5,024,939 and by Stillman et al. Mol. Cell. Biol. 5: 2051-2060 (1985). Preferred cells for heterologous protein expression are those that can be readily and efficiently transfected.

Cells used in the present invention can be from continuous cell lines or primary cell lines obtained from, for example, mammalian tissues, organs, or fluids. Tissue sections as well as disperse cells can be used in the present invention. Cells can also be obtained from transgenic animals that have been engineered to express a reporter gene. Cells obtained from transgenic or non-transgenic animals are preferred for cells that are difficult to culture *in vitro*, such as neural and hepatic cells. Primary cell lines can be made continuous using known methods, such as fusing primary cells with a continuous cell line or expressing transforming proteins. Cells of the invention can be stored or used with methods of the invention as isolated, clonal populations in plates such as those described in commonly owned United States Patent Applications, entitled "Low background multi-well plates and platforms for spectroscopic measurements" (Coassin et al., filed June 2, 1997); and entitled "Low background multi-well plates with greater than 864 wells for spectroscopic measurements" (Coassin et at., filed June 2, 1997).

Preferably, cells are stored or used in plates with 96, 384, 1536 or 3456 wells per plate. A single cell or a plurality of cells can be placed in such wells. Such isolated clonal populations will typically have 1,000, 10,000, or 100,000 or more such populations representative of substantially equivalent numbers of independent integrations sites. Such panels can be used in profiling, pathway identification, modulator identification, modulator characterization, and other methods of the invention.

Prior to being transfected with a trapping vector of the present invention, cells can be transfected with an exogenous gene capable of expressing an exogenous protein, such as a receptor (e.g., GPCR) or gene associated with the pathology of an etiological agent, such as a virus, bacteria, or parasite. Cells that express such exogenous proteins can then be transfected with a trapping vector to form a library of clones that can be screened The application also discloses animals with β-lacatmase expression constructs integrated into the genome of interest.

Many of the cells used can report modulation of biological processes by a variety of additional reporter genes or chemicals or combinations thereof. For example, beta-lactamase, an enzyme, can convert non-chromogenic substrates to chromogenic products or alter the chromogenic or fluorescent properties of a substrate such as CCF2. Furthermore, fluorescent reporters, such as fluorescent proteins, such as green fluorescent protein (GFP) molecules, can be used as reporters. Some mutant GFP molecules have different fluorescent properties as compared to wild-type GFP. These GFPs can be used as reporters and can be used singly or in combination with the present invention. For example, cells can have multiple reporters that can be differentiated to report different biological processes, or different steps within a biological process, such as steps in a signal transduction pathway.

### Targets

Proteins of interest that can be expressed in the cells of the invention include,: hormone receptors (e.g. mineralcorticosteroid, gluococorticoid, and thyroid hormone receptors); intracellular receptors (e.g., orphans, retinoids, vitamin D3 and vitamin A receptors); signaling molecules (e.g., kinases, transcription factors, or molecules such signal transducers and activators of transcription) (Science Vol. 264, 1994, p.1415-1421; Mol. Cell Biol., Vol. 16, 1996, p.369-375); receptors of the cytokine superfamily (e.g. erthyropoietin, growth hormone, interferons, and interleukins (other than IL-8) and colony-stimulating factors); G-protein coupled receptors, see US patent 5,436,128 (e.g., for hormones, calcitonin, epinephrine, gastrin, and pancrine or autocrine mediators, such as stomatostatin or prostaglandins) and neurotransmitter receptors (norepinephrine, dopamine, serotonin or acetylcholine); tyrosine kinase receptors (such as insulin growth factor, nerve growth factor (US patent 5,436,128)). Examples of the use of such proteins is further described herein.

Any target, such as an intracellular or extracellular receptor involved in a signal transduction pathway, such as the leptin or GPCR pathways, can be used with the present invention. Furthermore, the genes activated or repressed by a target can be isolated, identified, and modulators of that gene identified using the present invention. For example, the present invention can identify a G-protein coupled receptor (GPCR) pathway, determine its function, isolated the genes modulated by the GPCR, and identify modulators of such GPCR modulated proteins.

As an introduction to GPCR cell biology, the activation of Gα₁₅ or Gα₁₆ can, through a G-protein signaling pathway, activate PLCβ, which in turn increases intracellular calcium levels. An increase in calcium levels can lead to modulation of a "calcium-responsive" promoter that is part of a signal transduction detection system, i.e., a promoter that is activated (e.g., a NFAT promoter AP-1) or inhibited by a change in calcium levels. One example of an NFAT DNA binding site is described in Shaw, et al. Science 291:202-205 (1988). Likewise, a promoter that is responsive to changes in protein kinase C levels (e.g., a "protein kinase C-responsive promoter") can be modulated by an active Gα protein through G-protein signaling pathway. Selected cells described herein can also include a G-protein coupled receptor. Genes encoding numerous GPCRs have been cloned (Simon et al., Science 252:802-808 (1991)), and conventional molecular biology techniques can be used to express a GPCR on the surface of a cell of the invention. Preferably, the sum responsive promoter can allow for only a relatively short lag (e.g., less than 90 minutes) between engagement of the GPCR and transcriptional activation. A preferred responsive promoter includes the nuclear factor of activated T-cell promoter (Flanagan et al., Nature 352:803-807 (1991)). Polynucleotides identified by methos of the invention can be used as response elements that are sensitive to intracellular signals (signal-response elements). Signal response elements can be used in the assays described herein, such as identification of useful chemicals. Such signal response elements may sensitive intracellular signals that include voltage, pH, and intracellular levels of Ca⁺⁺, ATP, ADP, cAMP, GTD, GDP, K⁺, Na+, Zn++, oxygen, metabolites and IP3.

In one aspect, cells can be transformed to express an exogenous receptor, such as GPCR. Such a transduced cell line can than be futher transduced with a trapping vector to make a library of clones that can be used to identify cells that report modulation of the exogenous receptor. Preferably, the host cell line would not appreciably express the exogenous receptor.

Based on the unique structure of GPCRs, which have seven hydrophobic, presumably trans-membrane, domains (see, Watson and Arkinstall, The G-Protein Linked Receptor Facts Book, Academic Press, New York (1994)) orphan GPCRs (GPCRs having no known function) can be identified by searching sequence databases, such as those provided by the National Library of Medicine (Bethesda, MD), for similar motifs and homologies. This same strategy can, of course, be used for any target, especially when a paradigm sequence or motif has been determined.

### Drug Discovery for Viruses and Other Pathogens

The function of genes from viruses or other pathogens that effect the expression of genes in cells, such as mammalian cells, can be determined using the present invention. Furthermore, chemicals that modulate these genes can be identified using the methods of the present invention. For example, many transforming viruses, after infecting a cell, have the effect of up-regulating genes involved in cell proliferation, which allows the virus-infected cells to produce additional viruses, which can infect additional cells. These transforming viruses can act by stimulating a receptor from the target cell. One example of the mechanism is the Friend Erythroleukemia virus. This virus uses the erythropoetin receptor for entry into the cells. When the virus is bound to the receptor, a pathway is activated that causes an over-proliferation of red blood cells. If the activation of the erythropoetin receptor is inhibited, a decrease in the accumulation of red blood cells would result which can prevent or reduce the severity of the leukemia. The development of an assay that reports the activation of mammalian target genes allows the identification of modulators of other viral or pathogenic dependent pathways. These modulators can be used as therapeutic agents.

A general procedure for establishing this assay uses the virus or an isolated viral protein as the stimulus for modulating a pathway. First, a gene-trapping library is made using a cell line that can be infected by the virus or activated by the viral protein. The virus is added to these cells, and clones are isolated that responded specifically to the viral infection by the expression of a reporter gene.

As an example, the GP-120 portion of HIV protein is known to have mitogenic effect on cells exposed to GP-120, which indicates that downstream signaling pathways are being activated that can be associated with the cytotoxicity of the virus and allow its proliferation. Cell clones can be isolated that are induced by this activation which can be used to screen for modulators of this cytotoxic or proliferative effect. Other viral proteins, such as NEF from HIV, can be used. Chemicals that inhibit this effect can have useful therapeutic value to treat viral infection or toxicity.

This approach can be applied to any cellular pathogen that has an effect on a target cells, such as cytotoxicity, cell proliferation, inflammation or other responses. Other etiological targets include other viruses, such as retroviruses, adenovirus, papillomavirus, herpesviruses, cytomegalovirus, adeno associated viruses, hepatitis viruses, and any other virus. In addition to viruses, any other pathogen, such as parasites, bacteria, and viroids, can be used in the present invention. Particular viral targets include, but are not limited to, NEF, Hepatitis X protein, and other viral proteins, such as those that can be encoded or carried by a virus. In addition, two or more viral components can be added to identify coviral pathogensis components. This is a particularly valuable tool for identifying pathways modulated by two or more viruses concurrently, or over time as in slow activating viral conditions. For example, cotransfection with HIV and CMV may be used. Viral targets or components do not include oncogenes or proto-oncogenes found in uninfected genomes, and gene products thereof.

### Screening Test Chemicals Using Portions Of The Genome

Cells comprising β-lactamase polynucleotides integrated in the genome can be contacted with test chemicals or modulators of a biological process and screened for activity. Usually, the test chemical being screened will have at least one defined target, usually a protein. The test chemical is normally applied to the cells to achieve a final predetermined concentration in the medium bathing the cells. Typically, screens are conducted at concentrations 100 uM or less, preferably 10 uM or less and preferably 1 uM or less for confirmatory screens. As described more fully herein, cells can be subjected to multiple rounds of screening and selection using the same chemical in each round to insure the identification of clones with the desired response to a chemical or with different chemicals to characterize which chemicals produce a response (either an increase or decrease β-lactamase activity) in the cells. Such methods can be applied to any chemical that alters the function of any the proteins mentioned herein or known in the art.

Chemicals and physiological processes without a defined target, however, can also be used and screened with the cells of the invention. For example, once a clone is identified as containing an active genomic polynucleotide that is activated by a particular cellular signal (including extracellular signals), for instance by a neurotransmitter, that same clone can be screened with chemicals lacking a defined target to determine if activation by the neurotransmitter is blocked or enhanced by the chemical. This is a particularly useful method for finding therapeutic targets downstream of receptor activation (in this case a neurotransmitter). Such methods can be applied to any chemical that alters the function of any the proteins mentioned herein or known in the art. This type of "targetless" assay is particular useful as a screening tool for the medial conditions and pathways described herein.

The methods and compositions described herein offer a number of advantages over the prior art. For instance, screening of mammalian based gene integration libraries is limited by the use of existing reporter systems. Many enzymatic reporter genes, such as secreted-alkaline phosphatase, and luciferase, cannot be used to assay single living cells (including FACS) because the assay requires cell lysis to determine reporter gene activity. Alternatively, β-galactosidase can detect expression in single cells but substrate loading requires permeabilization of cells, which can cause deleterious effects on normal cell functions. Additionally, the properties of fluorescent β-galactosidase substrates, such as fluoroscein di-β-D-galactopyranside, and products make it very difficult to screen large libraries for both expressing and non-expressing cells because the substrate and product is not well retained or permits ratiometric analysis to determine the amount uncleaved substrate. Green fluorescent protein (GFP), a non-enzymatic reporter, could be used to detect expression in single living cells but has limited sensitivity. GFP expression level would have to be at least 100,000 molecules per cell to be detectable in a screening format and small changes in, or low levels of, gene expression could not be measured. Furthermore GFP is relatively stable and would not be suitable for measuring down-regulation of genes. Other advantages of the invention are described herein or readily recognized by one skilled in the art upon reviewing this disclosure.

### Methods for Rapidly Identifying Modulators of Genomic Polynucleotides

The invention provides for a method of identifying proteins or chemicals that directly or indirectly modulate a genomic polynucleotide. Generally, the method comprises inserting a β-lactamase expression construct into an eukaryotic genome, usually non-yeast, contained in at least one living cell, contacting the cell with a predetermined concentration of a modulator, and detecting β-lactamase activity in the cell. Preferably, cleavage of a membrane permeant β-lactamase substrate is measured and the membrane permeant β-lactamase substrate is transformed in the cell into a trapped substrate. Preferably, the β-lactamase expression construct comprises a β-lactamase polynucleotide, a splice donor, a splice acceptor and an IRES element. The method can also include determining the coding nucleic acid sequence of a polynucleotide operably linked to the β-lactamase expression construct using techniques known in the art, such as RACE.

### Modulator Identification

Modulators described herein can be used in this system to test for an increase or decrease in β-lactamase activity in successfully integrated clones. Such cells can optionally include specific proteins of interest as discussed herein. For example, the cell can include a protein or receptor that is known to bind the modulator (e.g., a nuclear receptor or receptor having a transmembrane domain heterologously or homologously expressed by the cell). A second modulator can be added either simultaneously or sequentially to the cell or cells and β-lactamase activity can be measured before, during or after such additions. Cells can be separated on the basis of their response to the modulator (e.g. responsive or non-responsive) and can be characterized with a number of different modulators to create a profile of cell activation or inhibition.

β-lactamase activity will often be measured in relation to a reference sample, often a control. For example, β-lactamase activity is measured in the presence of the modulator and compared to the β-lactamase activity in the absence of the modulator or possibly a second modulator. Alternatively, β-lactamase activity is measured from a cell expressing a protein of interest and to a cell not expressing the protein of interest (usually the same cell type). For instance, a modulator may be known to bind to a receptor expressed by the cell and the β-lactamase activity in the cell is increased in the presence of the modulator compared to the β-lactamase activity detected from a corresponding cell in the presence of the modulator, wherein the corresponding cell does not express the receptor.

### Pathway Identification and Modulators

When a reporter gene integrates into the genome of a host cell such that the reporter gene is expressed under a variety of circumstances, these clones can be used for drug discovery and functional genomics. These clones report the modulation of the reporter gene in response to a variety of stimuli, such as hormones and other physiological signals. These stimuli can be involved in a variety of known or unknown pathways that are modulated by known or unknown modulators or targets. Thus, these clones can be used as a tool to discover chemicals that modulate a particular pathway or to determine a cellular pathway.

These pathways are quite varied, and fall into general classes, which have specific species, which can be modulated by known or unknown modulators or agonists or antagonists thereof. By way of example, **Table 1** illustrates various pathways, species of these classes, and known modulators of these species. The invention can be used to identify regions of the genome that are modulated by such pathways, or physiological event

**TABLE 1**

| **Pathways and modulators** | | |
|---|---|---|
| **Pathway/Physiological Event** | | |
| **Genus** | **Species** | **Known Modulator** |
| Nuclear receptors | Estrogen receptor | Estrogen |
| Cytokines | IL-2 receptor | IL-2 |
| GPCRs | Vasopressin receptor | Vasopressin |
| Transcription factors | Fos or Jun | NFAT |
| Kinase dependent | Protein kinase C | PMA |
| Phosphatase dependent | Calcineurine | Cyclosporin A |
| Protease dependent | Metalloprotease | TIMPs |
| Chemokine | CCR I | RANTES |
| Ion channels | Calcium channels | Many known blockers |
| Second messenger dependent | Cyclic AMP | CAMP inhibitor protein |
| Cell differentiation | Hematopoeitic development | EPO |
| Cell growth | IL-2 receptor | IL-2 |
| Cell cycle dependent | CDK | P21 |
| Apoptosis | Fas | P53 |

In one embodiment, a genomic assay system to identify downstream transcriptional targets for signaling pathways is provided. This method requires the target of interest to activate gene expression upon addition of chemical or expression of the target protein. A cell line that is the most similar to the tissue type where the target functions is preferred for generating a library of clones with different integration sites with β-lactamase polynucleotides or other reporter genes. This cell line may be known to elicit a cellular response, such as differentiation upon addition of a particular modulator. If this type of cell line is available, it is preferred for screening, as it represents the native context of the target. If a cell line is not available that homologously expresses the target; a cell line can be generated by heterologously expressing the target in the most relevant cell line. For instance, if the target is normally expressed in the lymphoid cells, then a lymphoid cell line would be used generate the library.

The library of clones, as described further herein, can be separated into two pools by FACS using the FRET system described herein: an expressing pool (e.g. blue cells) and a non-expressing pool (e.g. green cells). These two pools can then be treated with a modulator followed by FACS to isolate induced clones (e.g. green to blue) or repressed clones (e.g. blue to green). Additional rounds of stimulation followed by FACS can be performed to verify initial results. The specificity of activation can be tested by adding additional chemicals that would not activate the defined target. This would allow the identification of clones that have β-lactamase polynucleotides integrated into genes activated by a variety of cellular signals.

Once a pool of cells with the desired characteristics are isolated they can be expanded and their corresponding genes cloned and characterized. Targets which could be used in this assay system include receptors, kinases, protein/protein interactions or transcription factors and other proteins of interest discussed herein.

In another embodiment, a method of identifying developmentally or tissue specific expressed genes is provided. β-lactamase polynucleotide can be inserted, usually randomly, into any precursor cell such as an embryonic or hematopoetic stem cell to create a library of clones. Constitutively expressing clones can be collected by sorting for blue cells and non-expressing cells collected by sorting for green cells using the FRET system described herein. The library of clones can then be stimulated or allowed to differentiate, and induced or repressed clones isolated. Cell surface markers in conjunction with fluorescent tagged antibodies or other detector molecules could be used to monitor the expression of reference genes simultaneously. Additionally, by stimulation and sorting stem cells at various developmental stages, it is possible rapidly identify genes responsible for maturation and differentiation of particular tissues.

Additionally, clones that have a β-lactamase polynucleotide integrated, either randomly or by homolgous recombination, into developmentally expressed genes can be used with FACS to isolate specific cell populations for further study, such as screening. Such methods can be used for identifying cell populations that have stem cells properties, as well as providing an intracellular reporter that allows isolation and screening of such a population of cells.

The present application can yield screening cell lines for a variety of targets whose downstream signaling elements are already known or postulated. These screening cell lines can be used to either screen for modulators of transfected targets or as readouts for expression cloning or functional analysis of uncharacterized targets. Screening cell lines can be made for any pathway or any modulator, such those described in **Table 1**.

In the case of ion channels, cell lines are generated in which β-lactamase expression is used to detect a voltage change. This is possible because intracellular signaling is sensitive to membrane potential and will modulate the expression of a subset of genes. In one example, a library of neuronal cells prepared following the general methods set forth in Examples 1 to 13, such as a dorsal root neuroblastoma cells, be screened for a response to a depolarization by incubating cells in high potassium (high K⁺) medium. Depending on the particular characteristics of the cell library and the method used, clones with a transcriptional response to a depolarizing treatment are identified by sorting for cells which changed from either green to blue or blue to green after depolarization. These clones are designated as voltage-sensitive clones and can be used as screening cell lines to identify chemicals that modulate ion channels (either endogenously expressed or transfected) which cause a voltage change upon either activation or inhibition (e.g. K⁺ or Na⁺ channels). These cells are also useful for expression cloning of ion channels. For example, a voltage-sensitive clone could be transfected with a cDNA library. Those cells transfected with functional channels that shift the membrane potential are detected via beta-lactamase and the cDNA gene products are analyzed for activity as ion channels.

Furthermore, a gene encoding a known ion channel can be transfected into the voltage sensitive cell line and then used as a screen for channel modulators. For example, expression or pharmacological activation of a Na⁺ channel can cause a depolarization that can be reported by the cell line. This cell line can be used to screen for agonists or antagonists, depending on the experimental protocol of ion channel modulators. In a variation of this approach, a genomic library from a cell line lacking K⁺ channels, such as L929 cells, can be directly transfected with a K⁺ channel gene. The expression of the K⁺ channel causes a voltage shift, such as a hyperpolarization, causing a change in expression of certain voltage-sensitive genes. The clones expressing these genes can be used to screen for regulators of the ion channel.

### Orphan protein signaling pathway identification and orphan protein modulators

In another embodiment, a method of identifying modulators of orphan proteins or genomic polynucleotides that are directly or indirectly modulated by an orphan protein is provided. Human disease genes are often identified and found to show little or no sequence homology to functionally characterized genes. Such genes are often of unknown function and thus encode for an "orphan protein." Usually such orphan proteins share less than 25% amino acid sequence homology with other known proteins or are not considered part of a gene family. With such molecules there is usually no therapeutic starting point. By using libraries of the herein described clones, one can extract functional information about these novel genes.

Orphan proteins can be expressed, preferably overexpressed, in living mammalian cells. By inducing over expression of the orphan gene and monitoring the effect on specific clones one may identify genes that are transcriptionally regulated by the orphan protein. By identifying genes whose expression is influenced by the novel disease gene or other orphan protein one may predict the physiological bases of the disease or function of the orphan molecule. Insights gained using this method can lead to identification of a valid therapeutic target for disease intervention.

### Modulator Identification using Genomic Polynucleotides Activated by Cellular Signals

In another embodiment, a method of screening a defined target or modulator using genomic polynucleotides identified with the methods described herein is provided. The gene identification methods described herein can also be used in conjunction with a screening system for any target that functions (either naturally or artificially) through transcriptional regulation.

In many instances a receptor and its ligand are known but not the downstream biological processes required for signaling. For example, a cytokine receptor and cytokine may be known but the downstream signaling mechanism is not. A library of clones generated from a cell line that expresses the cytokine receptor can be screened to identify clones showing changes in gene expression when stimulated by the cytokine. The induced genes could be characterized to describe the signaling pathway. Using the methods of the invention, gene characterization is not required for screen development, as identification of a cell clone that specifically responds to the cytokine constitutes a usable secondary screen. Therefore, clones that show activation or deactivation upon the addition of the cytokine can be expanded and used to screen for agonists or antagonists of cytokine receptor. The advantage of this type of screening is that it does not require an initial understanding of the signaling pathway and is therefore uniquely capable of identifying leads for novel pathways.

In another embodiment, a method of functionally characterizing a target using a panel of clones having active genomic polynucleotides as identified herein is provided. As large numbers of specifically responding cell lines containing active genomic polynucleotides identified with a particular biological process or modulator are generated, panels containing specific clones can be used for functional analysis of other potential cellular modulators. These panels of responding cell lines can be used to rapidly profile potential transcriptional regulators. Such panels, as well as containing clones with identified active genomic polynucleotides, which were generated by the invention panels, can include clones generated by more traditional methods. Clones can be generated that contain both the identified active genomic polynucleotide with a β-lactamase polynucleotide and specific response elements, such as SRE, CRE, NFAT, TRE, IRE, or reporters under the control of specific promoters. These panels would therefore allow the rapid analysis of potential effectors and their mechanisms of cellular activation. A second reporter (e.g. β-galactosidase gene can also be used with this method, as well as the other method described herein.

In another embodiment, a method of test chemical profiling using a clone or panel of clones having identified active polynucleotides is provided. Test chemical characterization is similar to target characterization except that the cellular target(s) do not have to be known. This method will therefore allow the analysis of test chemical (e.g. lead drugs) effects on cellular function by defining genes effected by the drug or drug lead.

Such a method can find application in the area of drug discovery and secondary affects (e.g. cytotoxic affect) of drugs. The potential drug would be added to a library of genomic clones and clones which either were induced or repressed would be isolated, or identified. This method is analogous to target characterization except that the secondary drug target is unknown. As well as providing a screen for the secondary effects, the assay provides information on the mechanism of toxicity.

### Methods Related to FACS and Identifying Active Genomic Polynucleotides

A method of identifying active genomic polynucleotides using clones having integrated β-lactamase polynucleotides and FACS is provided β-lactamase integration libraries can be used in a high-throughput screening format, such as FACS, to detect transcriptional regulation. The compatibility of β-lactamase assays with FACS enables a systematic method for defining patterns of transcriptional regulation mediated by a range of factors. This approach has not been feasible or practical using existing reporter systems. This new method will allow rapid identification of genes responding to a variety of signals, including tissue specific expression and during pattern formation.

For example, after integration of a β-lactamase polynucleotide, expressing and non-expressing cells can be separated by FACS. These two cell populations can be treated with potential modulators and changes in gene expression can be monitored using ratio-metric fluorescent readout. Pools of clones will be isolated that show either up- or down-regulation of reporter gene expression. Target genes from responding clones can then be identified. In addition, by being able to separate expressing and non-expressing cells at different time points after modulator addition, genes which are differentially regulated over time can be identified. This approach therefore enables the elucidation of transcription cascades mediated by cellular signaling. Specifically, it will provide a means to identify downstream genes which are transcriptionally regulated by a variety of molecules including, nuclear receptors, cytokine receptors or transcription factors.

Applications of this technology are nearly unlimited in the areas of gene discovery and functional analysis. Libraries of cell lines from various tissue types could be generated and used to identify genes with specific expression patterns or regulation mechanisms. These libraries of clones would represent millions of integration sites saturating the genome and can permit the identification of any expressed gene based on its transcriptional regulation. The features of the β-lactamase reporter system, in part, allow its use for this genomic integration assay in a high-throughput format

There are a variety of other approaches that may be used with the invention, including approaches similar to those proposed for β-lactamase. Examples would include antibody epitopes presented on the cell surface with fluorescent antibodies to detect positive cells. Gel matrixes could also be used which retain secreted reporters and allow detection of positive cells. These approaches would, however, be limited in sensitivity and would not be ratiometric in their detection. They would therefore allow for only the sorting of positive cells based on fluorescent intensity.

Once active genomic polynculeotides have been identified, they can be sequenced using various methods, including RACE (rapid amplification of cDNA ends). RACE is a procedure for the identification of unknown mRNA sequences that flank known mRNA sequences. Both 5' and 3' ends can be identified depending on the RACE conditions. 5' RACE is done by first preparing RNA from a cell line or tissue of interest. This total or polyA RNA is then used as a template for a reverse transcription reactions which can either be random primed or primed with a gene-specific primer. A poly nucleotide linker of known sequence is then attached to the 3' end of the newly transcribed cDNA by terminal transferase or RNA ligase. This cDNA is then used as the template for PCR using one primer within the reporter gene and the other primer corresponding to sequence which had been linked to the 3' end of the first stand cDNA. The present application is particularly well suited for such techniques and does not require construction of additional clones or constructs once the genomic polynucleotide has been identified.

### Substrates for Measuring β-lactamase Activity

Any membrane permanent β-lactamase substrate capable of being measured inside the cell after cleavage can be used in the methods and compositions of the invention. Membrane permanent β-lactamase substrates will not require permeablizing eukaryotic cells either by hypotonic shock or by electroporation. Generally, such nonspecific pore forming methods are not desirable to use in eukaryotic cells because such methods injure the cells, thereby decreasing viability and introducing additional variables into the screening assay (such as loss of ionic and biological contents of the shocked or porated cells). Such methods can be used in cells with cell walls or membranes that significantly prevent or retard the diffusion of such substrates. Preferably, the membrane permeant β-lactamase substrates are transformed in the cell into a β-lactamase substrate of reduced membrane permeability (usually at least five less permeable) or that is membrane impermeant. Transformation inside the cell can occur via intracellular enzymes (e.g. esterases) or intracellular metabolites or organic molecules (e.g. sulfhydryl groups). Preferably, such substrates are fluorescent. Fluorescent substrates include those capable of changes, either individually or in combination, of total fluorescence, excitation or emission spectra or FRET.

Preferably, FRET type substrates are employed with the methods . Including fluorogenic substrates of the general formula I: D-S-A
wherein D is a FRET donor and A is a FRET acceptor and S is a substrate for a protein with β-lactamase activity. β-lactamase activity cleaves either D-S or S-A bonds thereby releasing either D or A, respectively from S. Such cleavage resulting from β-lactamase activity dramatically increases the distance between D and A which usually causes a complete loss in energy transfer between D and A. Generally, molecules of D-S-A structure are constructed to maximize the energy transfer between D and A. Preferably, the distance between D and A is generally equal to or less than the Rₒ.

As would readily be appreciated by those skilled in the art, the efficiency of fluorescence resonance energy transfer depends on the fluorescence quantum yield of the donor fluorophore, the donor-acceptor distance and the overlap integral of donor fluorescence emission and acceptor absorption. The energy transfer is most efficient when a donor fluorophore with high fluorescence quantum yield (preferably, one approaching 100%) is paired with an acceptor with a large extinction coefficient at wavelengths coinciding with the emission of the donor. The dependence of fluorescence energy transfer on the above parameters has been reported Forster, T. (1948) Ann. Physik 2: 55-75; Lakowicz, J. R., Principles of Fluorescence Spectroscopy, New York: Plenum Press (1983); Herman, B., Resonance energy transfer microscopy, in: Fluorescence Microscopy of Living Cells in Culture, Part B, Methods in Cell Biology, Vol. 30, ed. Taylor, D.L. & Wang, Y.L., San Diego: Academic Press (1989), pp. 219-243; Turro, N. J., Modern Molecular Photochemistry, Menlo Part: Benjamin/Cummings Publishing Co., Inc. (1978), pp. 296-361, and tables of spectral overlap integrals are readily available to those working in the field for example, Berlman, I.B. Energy transfer parameters of aromatic compounds, Academic Press, New York and London (1973). The distance between donor fluorophore and acceptor dye at which fluorescence resonance energy transfer (FRET) occurs with 50% efficiency is termed Rₒ and can be calculated from the spectral overlap integrals. For the donor-acceptor pair fluorescein - tetramethyl rhodamine which is frequently used for distance measurement in proteins, this distance Rₒ is around 50-70 A dos Remedios, C.G. et al. (1987) J. Muscle Research and Cell Motility 8:97-117. The distance at which the energy transfer in this pair exceeds 90% is about 45 A. When attached to the cephalosporin backbone the distances between donors and acceptors are in the range of 10 A to 20 A, depending on the linkers used and the size of the chromophores. For a distance of 20 A, a chromophore pair will have to have a calculated Rₒ of larger than 30 A for 90% of the donors to transfer their energy to the acceptor, resulting in better than 90% quenching of the donor fluorescence. Cleavage of such a cephalosporin by β-lactamase relieves quenching and produces an increase in donor fluorescence efficiency in excess of tenfold. Accordingly, it is apparent that identification of appropriate donor-acceptor pairs for use as taught herein in accordance with the present invention would be essentially routine to one skilled in the art.

Reporting gene substrates described in Tsien et al., PCT Publication No. WO96/30540 published October 3, 1996 are preferred for β-lactamase.

### Fluorescence Measurements

When using fluorescent substrates, it will recognized that different types of fluorescent monitoring systems can be used to practice the invention. Preferably, FACS systems are used or systems dedicated to high throughput screening e.g., 96 well or greater microtiter plates. Methods of performing assays on fluorescent materials are well known in the art and are described in, e.g., Lakowicz, J. R., Principles of Fluorescence Spectroscopy, New York: Plenum Press (1983); Herman, B., Resonance energy transfer microscopy, in: Fluorescence Microscopy of Living Cells in Culture, Part B, Methods in Cell Biology, vol. 30, ed. Taylor, D.L. & Wang, Y. L., San Diego: Academic Press (1989), pp. 219-243; Turro, N. J., Modern Molecular Photochemistry, Menlo Park: Benjamin/Cummings Publishing Col, Inc. (1978), pp. 296-361.

Fluorescence in a sample can be measured using a fluorimeter. In general, excitation radiation, from an excitation source having a first wavelength, passes through excitation optics. The excitation optics cause the excitation radiation to excite the sample. In response, fluorescent proteins in the sample emit radiation that has a wavelength that is different from the excitation wavelength. Collection optics then collect the emission from the sample. The device can include a temperature controller to maintain the sample at a specific temperature while it is being scanned. According to one embodiment, a multi-axis translation stage moves a microtiter plate holding a plurality of samples in order to position different wells to be exposed. The multi-axis translation stage, temperature controller, auto-focusing feature, and electronics associated with imaging and data collection can be managed by an appropriately programmed digital computer. The computer also can transform the data collected during the assay into another format for presentation.

Preferably, FRET is used as a way of monitoring β-lactamase activity inside a cell. The degree of FRET can be determined by any spectral or fluorescence lifetime characteristic of the excited construct, for example, by determining the intensity of the fluorescent signal from the donor, the intensity of fluorescent signal from the acceptor, the ratio of the fluorescence amplitudes near the acceptor's emission maxima to the fluorescence amplitudes near the donor's emission maximum, or the excited state lifetime of the donor. For example, cleavage of the linker increases the intensity of fluorescence from the donor, decreases the intensity of fluorescence from the acceptor, decreases the ratio of fluorescence amplitudes from the acceptor to that from the donor, and increases the excited state lifetime of the donor.

Preferably, changes in the degree of FRET are determined as a function of the change in the ratio of the amount of fluorescence from the donor and acceptor moieties, a process referred to as "ratioing." Changes in the absolute amount of substrate, excitation intensity, and turbidity or other background absorbances in the sample at the excitation wavelength affect the intensities of fluorescence from both the donor and acceptor approximately in parallel. Therefore the ratio of the two emission intensities is a more robust and preferred measure of cleavage than either intensity alone.

The excitation state lifetime of the donor moiety is, likewise, independent of the absolute amount of substrate, excitation intensity, or turbidity or other background absorbances. Its measurement requires equipment with nanosecond time resolution, except in the special case of lanthanide complexes in which case microsecond to millisecond resolution is sufficient.

The ratio-metric fluorescent reporter system described herein has significant advantages over existing reporters for gene integration analysis, as it allows sensitive detection and isolation of both expressing and non-expressing single living cells. This assay system uses a non-toxic, non-polar fluorescent substrate that is easily loaded and then trapped intracellularly. Cleavage of the fluorescent substrate by β-lactamase yields a fluorescent emission shift as substrate is converted to product. Because the β-lactamase reporter readout is ratiometric it is unique among reporter gene assays in that it controls for variables such as the amount of substrate loaded into individual cells. The stable, easily detected, intracellular readout eliminates the need for establishing clonal cell lines prior to expression analysis. With the β-lactamase reporter system or other analogous systems flow sorting can be used to isolate both expressing and non-expressing cells from pools of millions of viable cells. This positive and negative selection allows its use with gene identification methods to isolate desired clones from large clone pools containing millions of cells each containing a unique integration site.

### High Throughput Screening System

The present application can be used with systems and methods that utilize automated and integratable workstations for identifying modulators, pathways, chemicals having useful activity and other methods described herein. Such systems are described generally in the art (see, U.S. Patent Nos: 4,000,976 to Kramer et al. (issued January 4, 1977), 5,104,621 to Pfost et al. (issued April 14, 1992), 5,125,748 to Bjornson et al. (issued June 30, 1992), 5,139,744 to Kowalski (issued August 18, 1992), 5,206,568 Bjornson et al. (issued April 27, 1993), 5,350,564 to Mazza et al. (September 27, 1994), 5,589,351 to Harootunian (issued December 31, 1996), and PCT Application Nos: WO 93/20612 to Baxter Deutschland GMBH (published October 14, 1993), WO 96/05488 to McNeil et al. (published February 22, 1996) and WO 93/13423 to Agong et al. (published July 8, 1993).

Typically, such a system includes: A) a storage and retrieval module comprising storage locations for storing a plurality of chemicals in solution in addressable wells, a well retriever and having programmable selection and retrieval of the addressable wells and having a storage capacity for at least 10,000 the addressable wells, B) a sample distribution module comprising a liquid handler to aspirate or dispense solutions from selected the addressable wells, the chemical distribution module having programmable selection of, and aspiration from, the selected addressable wells and programmable dispensation into selected addressable wells (including dispensation into arrays of addressable wells with different densities of addressable wells per centimeter squared), C) a sample transporter to transport the selected addressable wells to the sample distribution module and optionally having programmable control of transport of the selected addressable wells (including adaptive routing and parallel processing), D) a reaction module comprising either a reagent dispenser to dispense reagents into the selected addressable wells or a fluorescent detector to detect chemical reactions in the selected addressable wells, and. a data processing and integration module. The addressable wells should be made of biocompatable materials that are also compatible with the assay to be performed (see, U.S. Patent Application, "Systems and methods for rapidly identifying useful chemicals in liquid samples" (Stylli et al., filed May 16, 1997).

The storage and retrieval module, the sample distribution module, and the reaction module are integrated and programmably controlled by the data processing and integration module. The storage and retrieval module, the sample distribution module, the sample transporter, the reaction module and the data processing and integration module are operably linked to facilitate rapid processing of the addressable sample wells. Typically, devices of the invention can process about 10,000 to 100,000 addressable wells, which can represent about 5,000 to 100,000 chemicals, in 24-hour period. Cells clones generated using the present invention can be individually deposited into wells of a multi-well platform having any number of wells, such as 96, 864, 3456, or more. The cells in the wells can be cultured, stored, screened, and inventoried using such a system.

The present application is also directed to chemical entities and information (e.g., modulators or chemicals or databases biological activities of chemicals or targets) generated or discovered by operation of the application particularly chemicals and information generated using such systems.

### Pharmacology and Toxicity of Candidate Modulators

The structure of a candidate modulator identified by the invention can be determined or confirmed by methods known in the art, such as mass spectroscopy. For putative modulators stored for extended periods of time, the structure, activity, and potency of the putative modulator can be confirmed

Depending on the system used to identify a candidate modulator, the candidate modulator will have putative pharmacological activity. For example, if the candidate modulator is found to inhibit T-cell proliferation (activation) *in vitro,* then the candidate modulator would have presumptive pharmacological properties as an immunosuppressant or anti-inflammatory (see, Suthanthiran et al., Am. J. Kidney Disease, 28:159-172 (1996)). Such nexuses are known in the art for several disease states, and more are expected to be discovered over time. Based on such nexuses, appropriate confirmatory *in vitro* and *in vivo* models of pharmacological activity, as well as toxicology, can be selected. The methods described herein can also be used to assess pharmacological selectivity and specificity, and toxicity.

Once identified, candidate modulators can be evaluated for toxicological effects using known methods (see, Lu, Basic Toxicology, Fundamentals, Target Organs, and Risk Assessment, Hemisphere Publishing Corp., Washington (1985); U.S. Patent Nos: 5,196,313 to Culbreth (issued March 23, 1993) and U.S. Patent No. 5,567,952 to Benet (issued October 22, 1996). For example, toxicology of a candidate modulator can be established by determining *in vitro* toxicity towards a cell line, such as a mammalian i.e. human, cell line. Candidate modulators can be treated with, for example, tissue extracts, such as preparations of liver, such as microsomal preparations, to determine increased or decreased toxicological properties of the chemical after being metabolized by a whole organism. The results of these types of studies are often predictive of toxicological properties of chemicals in animals, such as mammals, including humans.

Alternatively, or in addition to these *in vitro* studies, the toxicological properties of a candidate modulator in an animal model, such as mice, rats, rabbits, or monkeys, can be determined using established methods (see, Lu, supra (1985); and Creasey, Drug Disposition in Humans, The Basis of Clinical Pharmacology, Oxford University Press, Oxford (1979)). Depending on the toxicity, target organ, tissue, locus, and presumptive mechanism of the candidate modulator, the skilled artisan would not be burdened to determine appropriate doses, LD₅₀ values, routes of administration, and regimes that would be appropriate to determine the toxicological properties of the candidate modulator. In addition to animal models, human clinical trials can be performed following established procedures, such as those set forth by the United States Food and Drug Administration (USFDA) or equivalents of other governments. These toxicity studies provide the basis for determining the efficacy of a candidate modulator *in vivo*. *Efficacy of Candidate Modulators*

Efficacy of a candidate modulator can be established using several art recognized methods, such as *in vitro* methods, animal models, or human clinical trials (see, Creasey, supra (1979)). Recognized *in vitro* models exist for several diseases or conditions. For example, the ability of a chemical to extend the life-span of HIV-infected cells *in vitro* is recognized as an acceptable model to identify chemicals expected to be efficacious to treat HIV infection or AIDS (see, Daluge et al., Antimicro. Agents Chemother. 41:1082-1093 (1995)). Furthermore, the ability of cyclosporin A (CsA) to prevent proliferation of T-cells *in vitro* has been established as an acceptable model to identify chemicals expected to be efficacious as immunosuppressants (see, Suthanthiran et al., supra, (1996)). For nearly every class of therapeutic, disease, or condition, an acceptable *in vitro* or animal model is available. Such models exist, for example, for gastro-intestinal disorders, cancers, cardiology, neurobiology, and immunology. In addition, these *in vitro* methods can use tissue extracts, such as preparations of liver, such as microsomal preparations, to provide a reliable indication of the effects of metabolism on the candidate modulator. Similarly, acceptable animal models may be used to establish efficacy of chemicals to treat various diseases or conditions. For example, the rabbit knee is an accepted model for testing chemicals for efficacy in treating arthritis (see, Shaw and Lacy. J. Bone Joint Surg. (Br) 55:197-205 (1973)). Hydrocortisone, which is approved for use in humans to treat arthritis, is efficacious in this model which confirms the validity of this model (see, McDonough, Phys. Ther. 62:835-839 (1982)). When choosing an appropriate model to determine efficacy of a candidate modulator, the skilled artisan can be guided by the state of the art to choose an appropriate model, dose, and route of administration, regime, and endpoint and as such would not be unduly burdened

In addition to animal models, human clinical trials can be used to determine the efficacy of a candidate modulator in humans. The USFDA, or equivalent governmental agencies, have established procedures for such studies.

### Selectivity of Candidate Modulators

The *in vitro* and *in vivo* method described above also establish the selectivity of a candidate modulator. It is recognized that chemicals can modulate a wide variety of biological processes or be selective. Panels of cells based on the present invention can be used to determine the specificity of the candidate modulator. Selectivity is evident, for example, in the field of chemotherapy, where the selectivity of a chemical to be toxic towards cancerous cells, but not towards non-cancerous cells, is obviously desirable. Selective modulators are preferable because they have fewer side effects in the clinical setting. The selectivity of a candidate modulator can be established *in vitro* by testing the toxicity and effect of a candidate modulator on a plurality of cell lines that exhibit a variety of cellular pathways and sensitivities. The data obtained from these *in vitro* toxicity studies can be extended animal model studies, including human clinical trials, to determine toxicity, efficacy, and selectivity of the candidate modulator.

The selectivity, specificity and toxicology, as well as the general pharmacology, of a test chemical can be often improved by generating additional test chemicals based on the structure/property relationships of the test chemical originally identified as having activity (a "Hit"). Test chemicals identified as having activity can be modified to improve various properties, such as affinity, life-time in the blood, toxicology, specificity and membrane permeability. Such refined test chemicals can be subjected to additional assays as described herein for activity analysis. Methods for generating and analyzing such chemicals are known in the art, such as U.S. patent 5,574,656 to Agrafiotis et al.

### Compositions

The present application also encompasses a modulator in a pharmaceutical composition comprising a pharmaceutically acceptable carrier prepared for storage and subsequent administration, which have a pharmaceutically effective amount of the candidate modulator in a pharmaceutically acceptable carrier or diluent. Chemicals identified by the methods described herein do not include chemicals publicly available as of the filing date of the present application or in the prior art. Acceptable carriers or diluents for therapeutic use are well known in the pharmaceutical art, and are described, for example, in Remington's Pharmaceutical Sciences, Mack Publishing Co. (A.R. Gennaro edit. 1985). Preservatives, stabilizers, dyes and even flavoring agents may be provided in the pharmaceutical composition. For example, sodium benzoate, sorbic acid and esters of p-hydroxybenzoic acid may be added as preservatives. In addition, antioxidants and suspending agents may be used.

The compositions of the present application may be formulated and used as tablets, capsules or elixirs for oral administration; suppositories for rectal administration; sterile solutions, suspensions for injectable administration; and the like. Injectables can be prepared in conventional forms either as liquid solutions or suspensions, solid forms suitable for solution or suspension in liquid prior to injection, or as emulsions. Suitable excipients are, for example, water, saline, dextrose, mannitol, lactose, lecithin, albumin, sodium glutamate, cysteine hydrochloride, and the like. In addition, if desired, the injectable pharmaceutical compositions may contain minor amounts of nontoxic auxiliary substances, such as wetting agents, pH buffering agents, and the like. If desired, absorption enhancing preparations (e.g., liposomes), may be utilized.

The pharmaceutically effective amount of the candidate modulator required as a dose will depend on the route of administration, the type of animal being treated, and the physical characteristics of the specific animal under consideration. The dose can be tailored to achieve a desired effect, but will depend on such factors as weight, diet, concurrent medication and other factors which those skilled in the medical arts will recognize. In practicing the methods, the pharmaceutical compositions can be used alone or in combination with one another, or in combination with other therapeutic or diagnostic agents. These products can be utilized *in vivo,* ordinarily in a mammal, preferably in a human, or *in vitro.* In employing them *in vivo,* the pharmaceutical composition can be administered to the mammal in a variety of ways, including parenterally, intravenously, subcutaneously, intramuscularly, colonically, rectally, nasally or intraperitoneally, employing a variety of dosage forms. Such methods may also be applied to testing chemical activity *in vivo*.

As will be readily apparent to one skilled in the art, the useful *in vivo* dosage to be administered and the particular mode of administration will vary depending upon the age, weight and mammalian species treated, the particular pharmaceutical composition employed, and the specific use for which the pharmaceutical composition is employed. The determination of effective dosage levels, that is the dosage levels necessary to achieve the desired result, can be accomplished by one skilled in the art using routine methods as discussed above. Typically, human clinical applications of products are commenced at lower dosage levels, with dosage level being increased until the desired effect is achieved. Alternatively, acceptable *in vitro* studies can be used to establish useful doses and routes of administration of the compositions identified by the present methods using established pharmacological methods.

In non-human animal studies, applications of potential pharmaceutical compositions are commenced at higher dosage levels, with the dosage being decreased until the desired effect is no longer achieved or adverse side effects are reduced or disappear. The dosage for the products of the present invention can range broadly depending upon the desired affects and the therapeutic indication. Typically, dosages may be between about 10 ng/kg and 1µg/kg body weight, preferably between about 100 µg/kg and 10 mg/kg body weight. Administration is preferably oral on a daily basis.

The exact formulation, route of administration and dosage can be chosen by the individual physician in view of the patient's condition. (see e.g., Fingl et al., in The Pharmacological Basis of Therapeutics, 1975). It should be noted that the attending physician would know how to and when to terminate, interrupt, or adjust administration due to toxicity, organ dysfunction, or other adverse effects. Conversely, the attending physician would also know to adjust treatment to higher levels if the clinical response were not adequate (precluding toxicity). The magnitude of an administrated dose in the management of the disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual patient. A program comparable to that discussed above may be used in veterinary medicine.

Depending on the specific conditions being treated, such pharmaceutical compositions may be formulated and administered systemically or locally. Techniques for formulation and administration may be found in Remington's Pharmaceutical Sciences, 18th Ed., Mack Publishing Co., Easton, PA (1990). Suitable routes may include oral, rectal, transdermal, vaginal, transmucosal, or intestinal administration; parenteral delivery, including intramuscular, subcutaneous, intramedullary injections, as well as intrathecal, direct intraventricular, intravenous, intraperitoneal, intranasal, or intraocular injections.

For injection, pharmaceutical compositions may be formulated in aqueous solutions, preferably in physiologically compatible buffers such as Hanks' solution, Ringer's solution, or physiological saline buffer. For such transmucosal administration, penetrants appropriate to the barrier to be permeated are used in the formulation. Such penetrants are generally known in the art. Use of pharmaceutically acceptable carriers to formulate the pharmaceutical compositions herein disclosed for the practice of the invention into dosages suitable for systemic administration is within the scope of the invention. With proper choice of carrier and suitable manufacturing practice, compositions, in particular, those formulated as solutions, may be administered parenterally, such as by intravenous injection. The pharmaceutical compositions can be formulated readily using pharmaceutically acceptable carriers well known in the art into dosages suitable for oral administration. Such carriers enable the chemicals to be formulated as tablets, pills, capsules, liquids, gels, syrups, slurries, suspensions and the like, for oral ingestion by a patient to be treated.

Agents intended to be administered intracellularly may be administered using techniques well known to those of ordinary skill in the art. For example, such agents may be encapsulated into liposomes, then administered as described above. All molecules present in an aqueous solution at the time of liposome formation are incorporated into the aqueous interior. The liposomal contents are both protected from the external microenvironment and, because liposomes fuse with cell membranes, are efficiently delivered into the cell cytoplasm. Additionally, due to their hydrophobicity, small organic molecules may be directly administered intracellularly.

Pharmaceutical compositions suitable for use include compositions wherein the active ingredients are contained in an effective amount to achieve its intended purpose. Determination of the effective amount of a pharmaceutical composition is well within the capability of those skilled in the art, especially in light of the detailed disclosure provided herein. In addition to the active ingredients, these pharmaceutical compositions may contain suitable pharmaceutically acceptable carriers comprising excipients and auxiliaries which facilitate processing of the active chemicals into preparations which can be used pharmaceutically. The preparations formulated for oral administration may be in the form of tablets, dragees, capsules, or solutions. The pharmaceutical compositions may be manufactured in a manner that is itself known, e.g., by means of conventional mixing, dissolving, granulating, dragee-making, levitating, emulsifying, encapsulating, entrapping, or lyophilizing processes. Pharmaceutical formulations for parenteral administration include aqueous solutions of the active chemicals in water-soluble form. Additionally, suspensions of the active chemicals may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents that increase the solubility of the chemicals to allow for the preparation of highly concentrated solutions.

Pharmaceutical compositions for oral use can be obtained by combining the active-chemicals with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dye-stuffs or pigments may be added to the tablets or dragee coatings for identification or to characterize different combinations of active chemical doses

### EXAMPLES

### Example 1 β-lactamase Expression Constructs

To investigate various beta-lactamase expression constructs **(BLECs)** multiple **BLECs** were constructed and transfected into mammalian cells.

The first of these, **BLEC-1** was constructed by cloning the cytoplasmic form of β-lactamase **SEQ.ID NO. 4** (see Table 2) such that it is functionally linked to the En-2 splice acceptor sequence, as shown in **FIG. 3**. This vector when inserted into a genomic intron will result in the generation of a fusion RNA between an endogenous target gene and β-lactamase ("BL"). **BLEC-1** also contains a bovine growth hormone poly-adenlyation sequence (BGH-polyA) downstream of the cytoplasmic Beta-lactamase.

**BLEC-2** was constructed identically to **BLEC-1**, except that a poliovirus internal ribosomal entry site (IRES) sequence was inserted between the En-2 splice acceptor β-lactamase("BL"). This eliminates reading frame restrictions and possible inactivation of beta-lactamase by fusion to an endogenous protein. To allow for selection of stable transfectants for **BLEC-1** and **BLEC-2** a neomycin or G418 resistance cassette was cloned downstream of the BGH poly-adenylation sequence. This cassette consists of a promoter, neomycin resistance gene and an SV40 poly- adenylation sequence, as shown in **FIG. 3**.

Two alternative constructs **BLEC-3** and **BLEC-4** were constructed similar to **BLEC-1,** and **BLEC-2** respectively, except the SV40-poly A was replaced with a splice donor sequence. This should enrich for insertion into transcribed regions, as it requires the presence of an endogenous splice acceptor and polyadenylation sequence downstream of the vector insertion site to generate G418 resistant clones. **BLEC-3** and **BLEC-4** also use the PGK promoter to drive the neomycin resistance gene instead of the human beta-actin promoter.

The structure of CCF2-AM (**BL** substrate) used in the experiments below is:

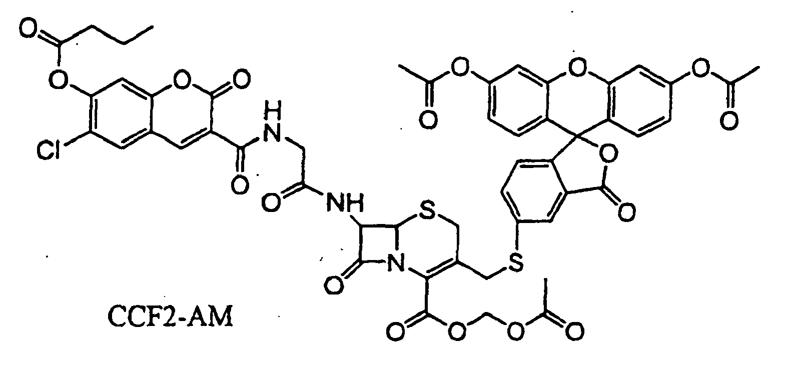

**Table 2**

| SEQ. ID NO. | parent -BL gene and reference | modification | mammalian expression vector | location of expression |
|---|---|---|---|---|
| # 1 | *Escherichia coli* RTEM Kadonaga et al. | Signal sequence replaced by: **ATG AGT** | pMAM-neo-glucocorticoid-inducible | cytoplasmic |
| # 2 | *Escherichia coli* RTEM Kadonaga et al. | Wild type secreted enzyme 2 changes in pre-sequence: ser 2 arg , ala 23 gly | pMAM-neo glucocorticoid-inducible | secreted extracellularly |
| # 3 | *Escherichia coli* RTEM | -globin up stream leader: AAGCTTTTTGCAGAAGCTCA GAATAAACGCAACTTTCCG | pCDNA 3 CMV promotor | cytoplasmic |
| | | Kozak sequence: | and | |
| | | GGTACCACCATGG signal sequence replaced by: **ATG GGG** | pZEO SV40 promoter | |
| # 4 | *Escherichia coli* RTEM | Kozak sequence: GGTACCACCATGG | pCDNA3 CMV promoter | cytoplasmic |
| | | signal sequence replaced by: | | |
| | | **ATG GAC** (GAC replaces CAT) | AND BLECs | |
| # 5 | *Bacillus licheniformis* 749/C Neugebauer et al. | signal sequence removed, new N-terminal ATG | pCDNA 3 CMV promotor | cytoplasmic |

**Table 3**

| **Functional Elements** | | | | | | |
|---|---|---|---|---|---|---|
| | Splice acceptor | Adapter | Reporter gene | Reporter gene poly A | Selection Promoter | Resistant Marker poly A |
| VECTORS | | | | | | |
| BLEC-1 | En2-splice acceptor | protein fusion | SEQ. ID NO. 4 | BGH polyA | β-actin promoter | Neo polyA |
| BLEC-2 | En2-splice acceptor | IRES | SEQ. ID NO. 4 | BGH polyA | β-actin promoter | Neo-polyA |
| BLEC-3 | En2-splice acceptor | Protein fusion | SEQ. ID NO. 4 | BGH polyA | PGK promoter | Neo-splice donor |
| BLEC-4 | En2-splice acceptor | IRES | SEQ. ID NO. 4 | BGH polyA | PGK promoter | Neo-splice donor |

### Example 2 Libraries of BLEC Clones

To investigate the function of each of the **BLEC** vectors they were transfected by electroporation into RBL-1 cells and stable clones were selected for each of the four **BLEC** plasmids (see **Table 2**). Selective media contained DMEM, 10% fetal bovine serum (FBS) and 400 µg/ml Geneticin (G418). G418 resistant cell clones were pooled from multiple transfections to generate a library of **BLEC** stable integrated clones.

This library of **BLEC-1** integrated clones was loaded with the fluorescent substrate of **BL** (CCF-2-AM) by adding 10µM CCF-2-AM in HBSS containing 10µM hepes 7. 1 and 1% glucose. After a 1 hour incubation at 22°C cells were washed with HBSS and viewed upon excitation with 400nm light using a 435nm long pass emission filter. Under these assay conditions 10% of the cells were blue fluorescent indicating they were expressing β-lactamase. This result suggests that that **BLEC-1** construct is functioning as a gene integration vector.

Stable cell lines were also generated by transfecting **BLEC-1** into **CHO-K1** and **Jurkat** cells. Populations of **BLEC-1** integrated clones from **CHO** and **Jurkat** cells showed similar results to those obtained with RBL-1 clones with 10-15 % of **BLEC** integrated cell clones expressing **BL** as determined by their blue/green ratio after loading with CCF-2-AM. This result shows that the **BLECs** function in a variety of cell types including human T-cells (Jurkat), rat basophilic leukocytes (RBL), and Chinese hamster ovarian (CHO).

### Example 3 Isolating BLEC Clones Expressing β-lactamase

Fluorescent activated cell sorting of multi-clonal populations of RBL-1 gene integrated clones was used to identify clones with regulated **BL** gene expression. A **BL** non-expressing population of cells was isolated by sorting a library of **BLEC-1** integrated clones generated by transfection of RBL-1 cells as described in **Example 2**. 180,000 clones expressing little or no **BL** were isolated by sorting for clones with a low blue/green ratio (R1 population), as shown in **FIG. 4A**. This population of clones was grown for seven days and resorted by FACS to test the population's fluorescent properties. FACS analysis of the cell clones sorted from R1 shows that most of the cells with a high blue/green ratio ∼0.1% have been removed by one round of sorting for green cells, as shown in **FIG. 4B**. It is also clear that the total population has shifted towards more green cells compared to the parent population, as shown in **FIG. 4A****.** There are, however, cells with a high blue/green ratio showing up in the green sorted population. These may represent clones in which the **BLEC** has integrated into a differentially regulated gene such as a gene whose expression changes throughout the cell cycle.

The population of RBL-1 clones shown in **FIG. 4B** was stimulated by addition of 1uM ionomycin for 6 hours and resorted to identify clones which had the **BLEC** integrated into a gene which is inducible by increasing intracellular calcium. Table 3 below summarizes the results from this experiment. A greater percentage of blue clones were present in all three of the blue sub-population (R4, R2, R5) in the ionomycin stimulated when compared to the unstimulated population. This sorted population represents the following classes of blue cells: R4 (highest blue/green ratio (bright blues)), R2 (multicolor blues), and R5 (lower blue/green ratio (least blue). Additionally, in the ionomycin stimulated population there is a decrease in the percent green cells from the unstimulated population (R6). This increase in blue clones in the ionomycin stimulated population indicates that a sub-population of blue clones have the **BLEC** inserted into a gene which is induced by ionomycin. Individual blue clones were sorted from the ionomycin stimulated population and are analyzed for their expression profile.

**Table 4**

| **Sort Window (See** **FIG. 4****)** | | | | |
|---|---|---|---|---|
| | R4 (blue) | R2 | R5 | R6 (green) |
| Unstimulated % | .11 | 2.39 | 1.53 | 66.23 |
| 1uM Ionomycin Stimulated % | .24 | 3.5 | 2.5 | 61.64 |
| Ratio +Ion/-Ion | 2.2 | 1.5 | 1.6 | .9 |

In addition to allowing the isolation of cell clones with inducible BL expression from large populations of cells, clones can be isolated based their level of **BL** expression. To isolate cells with different levels of **BL** expressions blue clones can be sorted after different exposure times to substrate or by their blue/green ratio. Cell with a lower blue/green ratio or those requiring longer incubation times will represent clones expressing lower levels of **BL.** This is demonstrated by the FACS scan above as clones sorted from the R4 window have a higher blue/green ration indicating they are expressing higher levels of **BL**, cells sorted from the R5 have a lower blue/green ratio (visually turquoise) indicating lower **BL** expression. Cell sorted from the R3 window which contain all the blue cells show variation in blue color from bright blue (high blue/green ratio) to turquoise blue (low blue/green ratio).

To demonstrate that the expression constructs are relatively stable for sorted clones cells were sorted from R3 (blue population) as shown in **FIG. 4A** and cultured in the absence of selective pressure for several weeks. There was little change in the percent of blue cells in the cultured population with the percent blue being maintained at ∼90%. This result represents a 10-fold enrichment for clones constitutively expressing **BL** by one round of FACS selection.

Cells in R6 window have the lowest blue/green ration and appear green visually. R6 cell is therefore not expressing **BL** or are expressing **BL** below the detection limit of our assay.

### Example 4 Stability of BLEC Clones

To further investigate the stability of reporter gene integrations into constitutively active genes, single blue clones were sorted from cell clone populations generated by transfecting RBL-1, and CHOK1 with **BLEC-1.** After addition of CCF-2 to the multi-clonal cell population, single blue clones were sorted into 96 well microtiter plates. These clones were expanded to 24 well dishes which took 7-10 days. The cell viability varied between the two cell types with 80% of the sorted clones forming colonies for the CHO and 36% for the RBL-1 cells. After expansion into a 24 well dishes 20 CHO **BLEC-1** stable clones were tested for **BL** expression by addition of CCF-2 -AM. 20/20 of these clones expressed **BL** with the percent blue cells within a clone ranging from 70% to 99%. This result is consisted with the earlier data presented for RBL-1 in which the blue sorted population was tested for **BL** expression after several weeks of non-selective culturing. There was however a significant differences between clones in their blue/green ratio and hence their level of **BL** expression. This suggested that genes with different levels of constitutive expression had been tagged with the **BLEC**. Although there was a significant differences in blue color between separate clones the blue fluorescence within a clone was consistently similar as would be expected in a clonal population. There were however green cells within the blue sorted clones, which may indicate that there is some loss of the **BLEC-1** plasmid integration site when clones are grown up from a single cell.

Single clones were expanded and used to make RNA for RACE to identify the target gene and DNA for southern analysis.

### Example 5 Isolation of Jurkat BLEC integrated clones that constitutively express beta-lactamase

Jurkat cells are a T-cell line derived from a human T-cell leukemia. This cell line maintains many of the signaling capabilities of primary T-cells and can be activated using anti-CD3 antibodies or mitogenic lectins such as phytohemaglutinin (PHA). Wild type Jurkat cells were transfected by electroporation with a beta-lactamase trapping construct (**BLEC-1, BLEC-1A**, or **BLEC-1B** see **FIG. 3**) ("**BLEC** constructs") that contains a gene encoding an beta-lactamase gene that is not under control of a promoter recognized by the Jurkat cells and a neomycin resistance gene that can be expressed in Jurkat cells. **BLEC-1** is set forth in **FIG. 3****. BLEC-1A** has a NotI site after the SV40 poly A site. This allows the cutting of the insert away form the plasmid backbone. **BLEC-1B** is the same as **BLEC-1A** except that the ATG at the beta-lactamase translation start has been changed to ATC. This eliminated the translation start site and requires the addition of an upstream ATG to produce beta-lactamase. Stable transformants were selected for their resistance to 800µg/ml G418. After 400 separate experiments, a pool of greater than one million clones with **BLEC** insertions was produced. This population of cells is a library of cell clones in which the **BLEC** construct inserted throughout the genome ("Jurkat **BLEC** library"). Approximately ten percent of the cells in this library express beta-lactamase in the absence of added stimuli. Beta-lactamase activity in the cells was determined by contacting the cells with CCF2-AM and loading in the presence of Pluronic 128 (from Sigma) at a about 100µg/ml. Individual clones or populations of cells that express beta-lactamase can be obtained by FACS sorting.

Genomic Southern analysis of these clones using a DNA probe encoding beta-lactamase showed the vector inserted into the host genome between one and three times per cell, with most clones having one or two vector insertion sites (for Genomic Southern analyses, see Sambrook, Molecular Cloning, A Laboratory Manual, Cold Spring Harbor Laboratory Press (1989)). Northern analysis of these clones using a DNA probe that encodes beta-lactamase showed that the level of expression and message size varied from clone to clone (for Northern analysis, see Sambrook, supra, (1989)). This indicated that fusion transcripts were being made with different genes functionally tagged with beta-lactamase, which allows for the reporter gene to be expressed under the same conditions as the endogenous gene. Using appropriate primers, RACE (Gibco BRL) was used to isolate the genes linked to the expressed beta-lactamase gene in a subset of these constitutively expressing clones. These genes were cloned and sequenced using known methods (see, Sambrook, supra, (1989)). These sequences were compared with known sequences using established BLAST search techniques. Known sequences that were identified included: beta-catenin, moesin, and β-adaptin. Additionally, several novel sequences were identified which represent putative genes.

### Example 6 Isolation of Jurkat BLEC integrated clones that show induced expression of beta-lactamase upon activation

Jurkat **BLEC** integrated clones that exhibit beta-lactamase expression upon activation of the Jurkat cells by PHA (PHA induced clones) were isolated by FACS sorting a Jurkat BLEC library. These clones represent cells in which the trapping construct had integrated into a gene up regulated by PHA (T-cell) activation. Thus, these cells report the transcriptional activation of a gene upon cellular activation. Individual clones were identified and isolated by FACS using CCF2-AM to detect beta-lactamase activity. This clone isolation method, the induced sorting paradigm, used three sequential and independent stimulation and sorting protocols. A FACS read out for Jurkat cells that don't contain a **BLEC** construct contacted with CCF2-AM was used as a control. These control cells were all green.

The first sorting procedure isolated a pool of blue (β-lactamase expressing, as indicated by contacting the cells with CCF2-AM) clones which had been pre-stimulated for 18 hours with 10µg/ml PHA from an unsorted Jurkat **BLEC** library. This pool represented 2.83 % of the original unsorted cell population. This selected pool contained clones that constitutively express beta-lactamase and clones in which the beta-lactamase expression was induced by PHA stimulation ("stimulatable clones"). After sorting, this pool of clones was cultured in the absence of PHA to allow the cells, in the case of stimulatable clones, to expand and return to a resting state (i.e. lacking PHA induced gene expression).

The second sorting procedure isolated a pool of green (non-β-lactamase expressing, as indicated by contacting the cells with CCF2-AM) cell clones from the first sorted pool that had been grown, post-sorting, without PHA stimulation for 7 days. The second sorting procedure separates clones that constitutively express beta-lactamase from cells that express beta-lactamase upon stimulation. This second pool represented 11.59% of the population of cells prior to the second sort. This pool of cells was cultured in the absence of PHA to amplify the cell number prior to a third sort.

The third sorting procedure used the same procedure as the first sorting procedure and was used to isolate individual cells that express beta-lactamase in response to being contacted with 10µg/ml PHA for 18 hours. Single blue clones were sorted individually into single wells of 96 well microtiter plates. This three round FACS sorting procedure enriched PHA inducible clones about 10,030 fold.

These isolated clones were expanded and tested for PHA inducibility by microscopic inspection with and without PHA stimulation in the presence of CCF2-AM. A total of fifty-five PHA inducible clones were identified using this procedure. The PHA inducibility for these clones ranged from a 1.5 to 40 fold change in the 460/530 ratio as compared to unstimulated control cells. Genomic Southern analysis using a DNA probe encoding beta-lactamase established that these clones represented 34 independent stable vector integration events. A list of clones obtained by the methods of the present invention and their characteristics is provided below in **Table 6** and **Table 7**.

In addition to PHA inducible clones, Phobol 12-myristate 13-acetate (PMA) (Calbiochem), Thapsigargin (Thaps) (Calbiochem), and PMA + Thaps inducible clones were isolated using the general procedure set forth above using the indicated inducer rather than PHA. PMA is a specific activator of PKC (protein kinase C) and Thaps is a specific activator of intracellular calcium ion release (Thaps). These clones were isolated using three rounds of FACS using the general procedures described for the PHA inducible clones in Example 5. In such instances, other stimulants were substituted for PHA. PMA was provided at 8 nM, Thaps was provided at 1µM. When these two stimulants were combined, their concentration was not changed. As shown in **Table 5,** clones were selected based on their activation by PMA, Thaps, or PMA with Thaps after three or eighteen hours of stimulation ("stimulation time"). These results demonstrate that the FACS sorting criteria can be varied depending upon the type of modulated clones desired. By using varied selection conditions, it is possible to isolate functionally distinct clones downstream of the desired signaling target.

### Example 7 Isolation of Jurkat BLEC integrated clones that show repressed expression of beta-lactamase upon activation

Jurkat **BLEC** clones that exhibit decreased beta-lactamase expression upon activation of the Jurkat cells by PHA were isolated by FACS sorting. These clones represent cells in which the BLEC trapping construct had integrated into a gene down regulated by PHA (T-cell) activation. Thus, these cells report the transcriptional repression of a gene upon cellular activation. Individual clones were identified and isolated by FACS using CCF2-AM to detect beta-lactamase activity using the following repressed sorting paradigm.

A first sort was used to isolate a population of cells that constitutively express beta-lactamase by identifying and isolating a population of blue cells from an unstimulated population of **BLEC** transfected Jurkat cells contacted with CCF2-AM. The sorted population of cells represented 2.89% of the unsorted population. These cells were cultured, divided into two pools, and stimulated with one of two different stimuli, either 10µg/ml PHA for 18 hours, or 8 nM PMA and 1µM Thapsigargin for 18 hours. These stimulated cells were contacted with CCF2 (loading in the presence of 400 PET (4% weight/volume) and Pluoronic^{®} 128 (100µg/ml)) and the green cells in the population were sorted using FACS. The sorted population represented 8.41 % of the cell population prior to the second sort. The third round of FACS was for single blue unstimulated cells. The population of cells obtained represented 18.2 % of the cell population prior to the third sort.

This sorting procedure represents a 2,260-fold enrichment for PHA repressible clones. These clones have the beta-lactamase gene integrated into a gene that is down regulated by PHA stimulation of the cells. Six of 80 individual clones tested were repressed by PHA or PMA + Thapsigargin. All of these clones were confirmed to be independent integration events by genomic Southern analysis using a DNA probe encoding beta-lactamase. The results of these studies are presented in **Table 5**.

**TABLE 5**

| **Identification of trapping cell lines with reporter genes expression which is regulated by T-cell activation** | | | | | | |
|---|---|---|---|---|---|---|
| Stimuli (Dose) | First Sort Activation Chemical and Time of Exposure | Stimulation Time | Sorting Paradigm | Clones isolated | Clones with One or Two Vector Insertion(s) | |
| | | | | | 1 | 2 |
| PHA (10µg/ml) | PHA 18 hours | 18 hours | Induced | 34 | 24 | 10 |
| PMA (8 nM) + Thaps (1 µM) | PMA + Thaps 3 hours | 3 hours | Induced | 2 | 2 | 0 |
| PMA (8 nM) | PMA 3 hours | 3 hours | Induced | 3 | 2 | 1 |
| Thaps (1µM) | Thaps 3 hours | 3 hours | Induced | 2 | 2 | 0 |
| PHA (10 g/ml) or PMA (8nm) + Thaps (1 µM) | No Stimulation | 18 hours | Repressed | 6 | 5 | 1 |

### Example 8 Specificity of T-cell modulated clones

isolated clones from PHA-induced (Example 6) and PHA-repressed (Example 7) procedures described above were characterized to determine the specificity of their modulation and time required for induction or repression. Clones were stimulated with multiple activators or inhibitors over a one to twenty-four hour time interval. As shown in **Table 6**, five clones produced by the induced and repressed sorting paradigms using a plurality of activators were tested for their responsiveness to a variety of T-cell activators, suppressors, and combinations thereof.

**TABLE 6**

| **Sorting protocols and specificity of activated BLEC Jurkat clones** | | | | | | | | | | | |
|---|---|---|---|---|---|---|---|---|---|---|---|
| Clone | Sorting Procedures | | | | Relative Beta-Lactamase Activity of the Clone by the Indicated Stimulus After 24 hours (% of maximum activated stimuli) | | | | | | |
| | Paradigm | First Sort Stimulus and (cell color sorted for) | Second Sort Stimulus and (cell color sorted for) | Third Sort Stimulus And (cell color sorted for) | None | PMA (8nM) | Thaps (1µM) | PMA (8nM) + Thaps (1µM) | PMA (8nM) + Thaps (1µM) + CsA (100 nM) | PHA (10 µg/ml) | PHA (10 µg/m) + CsA (100 nM) |
| J83-P19 | Induced | PHA' (blue) | N/S (green) | PHA (blue) | 0 | <1 | 100 | 50 | <5 | 60 | <5 |
| J32-6D4 | Induced | PHA (blue) | N/S (green) | PHA (blue) | 0 | 60 | 1-2 | 100 | 70 | 80 | 75 |
| C2 | N/S | N/S | N/S | N/S | 0 | <1 | 0 | 100 | <1 | 30 | 1 |
| J389-PT14 | Induced | PMA^{b} + Thaps^{c} (blue) | N/S (green) | PMA + Thaps (blue) | 0 | 90 | 5 | 85 | 100 | 85 | 90 |
| J83 97-PPTR2 | Repressed | N/S (blue) | PMA + Thaps (green) | N/S (blue) | 0 | 100 | 85 | 50 | 85 | 67 | 75 |
| J83-PTT8 | Induced | PHA (blue) | N/S (green) | PHA (blue) | 0 | 80 | 100 | 25 | 70 | 60 | 60 |

| | | | | | | | | | | | |
|---|---|---|---|---|---|---|---|---|---|---|---|
| "N/S" means "no stimulation" ^{a} concentration of PHA used was 10µg/ml. ^{b} concentration of PMA used was nM. ^{c} concentration of Thaps used was 1µM | | | | | | | | | | | |

In this study, PMA, which is a PKC activator, Thapsigargin which increases intracellular calcium, PHA which activates the T-cell receptor pathway, and cyclosporin A which is a clinically approved immunosuppressant that inhibits the Ca²⁺ dependent phosphates calcineurin were investigated for their ability to modulate beta-lactamase expression in PHA induced and repressed **BLEC** clones.

The selected clones show varied dependence for their activation and inhibition by these activators and inhibitors which give and indication of the signaling events required for their transcriptional activation. Five of the listed clones were generated using the approaches described above in Example 6. The clone C2 was generated using a more classical approach. This clone was generated by transfecting a plasmid construct in which a 3X NFAT response element has been operably linked to beta-lactamase expression. This 3XNFAT element represents a DNA sequence that is present in the promoter region of IL-2 and other T-cell activated genes. In addition the C2 cell line has been stably transfected with the M1 muscurinic receptor. This allows the activation of beta-lactamase expression in this clone using an M1-muscurinic agonist such as carbachol. This cell line therefore represents a good control for the cellular activators and inhibitors tested as the signaling events required for its activation are established.

The results of these studies indicate that the cell lines generated vary in their specificity towards activation or repression by activators. Thus, depending on the type of system that these cells are to be used to investigate, a panel of clones with varying specificity towards a specific pathway are made available by the present methods.

**Table 7** and **Table 8** provide data similar to that provided in **Table 5** for all of the clones obtained by the methods of Examples 5 to 7.

**TABLE 7**

| **Characterization of induced BLEC Jurkat clones** | | | | | | |
|---|---|---|---|---|---|---|
| | | Change in 460/530 ratio in the indicated clone by the following activator | | | | |
| CLONE Number | TIME (hours) for first detectable change in color | PHA (10µg/ml) | Thaps (1µM) | PMA (8 nM) | PMA (8 nM) + Thaps (1 µM) | Anti-CD3 (2µg/ml) (Pharmingen) |
| J32585 | 6 | 7 | Nt | 2-3 | Nt | 4-5 |
| J325B11 | 6 | 9 | 1-2 | 2-3 | Nt | 5-6 |
| J325E3 | 6 | 7 | Nt | 2-3 | Nt | 4-5 |
| J325G4 | 6 | 3-4 | Nt | 3-4 | Nt | 4-5 |
| J325E6 | 6 | 11 | Nt | 3-4 | Nt | 6 |
| J326C9 | 6 | 4-5 | 1-2 | 2-3 | Nt | 34 |
| J325E1 | <2 | 8 | Nt | 8 | Nt | 5-6 |
| J326D4 | <2 | 10 | 0 | 10 | Nt | 5-6 |
| J326D7 | <2 | 10 | Nt | 10 | Nt | 5-6 |
| J326F7 | <2 | 10 | Nt | 10 | Nt | 5-6 |
| J326H4 | <2 | 10 | Nt | 10 | Nt | 5-6 |
| J83PI1 | Nt | 3-4 | 3-4 | 3-4 | 4-5 | 2-3 |
| J83PI2 | 5-6 | 8 | 1-2 | 7-8 | 7-8 | 3-4 |
| J83PI8 | 5-6 | 4-5 | 1-2 | 4-5 | 4-5 | 2-3 |
| J83PI3 | 5-6 | 5-6 | 6-7 | 3-4 | 5-6 | 2-3 |
| J83PI4 | 4-6 | 3-4 | 3-4 | 0 | 2-3 | 2 |
| J83PI6 | 6-18 | 6-7 | 7-8 | 0 | 4-5 | 4 |
| J83PI9 | 6 | 6 | 5-6 | 0 | 4-5 | 3-4 |
| J83PI5 | Nt | Nt | Nt | Nt | Nt | Nt |
| J83PI7 | 6-18 | 2 | 2 | 2 | 2 | 1.5-2 |
| J83PI15 | Nt | 3-4 | 2 | 3-4 | 3-4 | 3-4 |
| J83PI16 | Nt | 3-4 | 1-2 | 3-4 | 3-4 | 2-3 |
| J83PI18 | Nt | 5-6 | 7-8 | 5 | Nt | Nt |
| J83PI12 | Nt | Nt | Nt | Nt | Nt | Nt |
| J83PI14 | Nt | 2 | 2 | 2 | Nt | Nt |
| J83PI17 | Nt | Nt | Nt | Nt | Nt | Nt |
| J83PI19 | Nt | 5-6 | 1-2 | 3 | 1-2 | 1-2 |
| J83PI11 | Nt | Nt | Nt | Nt | Nt | Nt |
| J83PI13 | Nt | 2-3 | 2-3 | 0 | Nt | Nt |
| J97PI1 | Nt | 3-4 | 3-4 | 3-4 | 3-4 | 3-4 |
| J97PI2 | Nt | 2-3 | Nt | Nt | 2-3 | Nt |
| J97PI3 | Nt | 1-2 | 1-2 | 1-2 | 1-2 | Nt |
| J97PI4 | Nt | 1-2 | 1-2 | 1-2 | 1-2 | Nt |
| J97PI5 | Nt | 1-5 | 1-9 | 1-5 | 2-3 | Nt |
| J97PI6 | Nt | 3-4 | 4-6 | 1-2 | 4-6 | Nt |
| J97PI13 | Nt | 2-3 | 5-6 | 1-2 | 4-5 | Nt |
| J97PI18 | Nt | 1-2 | 3-4 | 1-2 | 4-5 | Nt |
| J97PI7 | Nt | 3-4 | 4-5 | 1-2 | 5-6 | Nt |
| J97PI17 | Nt | 4-5 | 7-8 | 1-2 | 8-10 | Nt |
| J97PI8 | Nt | 2.5-3 | 3-4 | 1-2 | 3-4 | Nt |
| J97PI9 | Nt | 2-3 | 4-5 | 1-2 | 5-6 | Nt |
| J97PI10 | Nt | 3-4 | 3-4 | 1-2 | 4-5 | Nt |
| J97PI23 | Nt | 4-5 | 4-5 | 1-2 | 4-5 | 1-2 |
| J97PI11 | Nt | 3-4 | 5-6 | 2 | 4-5 | Nt |
| J97PI15 | Nt | 1-2 | 3-4 | 1-2 | 3-4 | Nt |
| J97PI12 | Nt | 3-4 | 5-6 | 2-3 | 5-6 | Nt |
| J97PI22 | Nt | 5-6 | 5-7 | 2-3 | 3-4 | 3-4 |
| J97PI14 | Nt | 4-5 | 3-4 | 2 | 4-5 | Nt |
| J97PI116 | Nt | 2-3 | 3-4 | 2-3 | 4 | Nt |
| J97PI19 | Nt | 2-3 | 2-3 | 1-2 | 2-4 | Nt |
| J97PI20 | Nt | 1-2 | 2-3 | 1-2 | 1-2 | Nt |
| J97PI21 | Nt | 2-3 | 2-3 | 1-2 | 2-3 | 2-3 |
| J97PI24 | Nt | 3-4 | 3-4 | 2-3 | 7-10 | 3-4 |
| J389PT1 | 2hours | 5-6 | 3-4 | 8-9 | 8-9 | 3-4 |
| J389PT4 | 1hour | 15 | 10 | 12 | 16 | 15 |
| J389PM2 | 1hour | 4-5 | 3-4 | 3-4 | 4-5 | 4-5 |
| J389PM3 | 1hour | 3-4 | 2-3 | 2-3 | 3-4 | 3-4 |
| J389PM5 | 1hour | 4-5 | 3-4 | 3-4 | 4-5 | 4-5 |
| J389PM7 | 3hours | 1-2 | 2-3 | 1-2 | 1-2 | 1-2 |
| J389PM8 | 2-3hours | 2-3 | 3-4 | 2-3 | 2-3 | 3-4 |
| J389TI1 | 3-5hours | 1-2 | 2-3 | 1-2 | 2-3 | 2-3 |
| J389TI4 | 2hour | 0 | 3-4 | 1-2 | 2-3 | 0 |

| | | | | | | |
|---|---|---|---|---|---|---|
| "Nt" means "not tested" | | | | | | |

**TABLE 8**

| **Characterization of repressed BLEC Jurkat clones** | | | | |
|---|---|---|---|---|
| | **Relative repression of beta-lactamase in the indicated clone by the following activator** | | | |
| **CLONE #** | **PHA (10 µg/ml)** | **PHA (10 µg/ml) + CsA (100 nM)** | **PMA (8 nMl) + Thaps (1 µM)** | **PMA (8 nM) + Thaps (1 µM) CsA (100 nM)** |
| J83/97pptr1 | 90 | 90 | 75 | 75 |
| J83/97 pptr2 | 10 | -60 | 10 | -80 |
| J83/97pptr3 | 10 | -50 | 10 | -100 |
| J83/97pptr4 | 60 | 60 | 40 | 70 |
| J83/97pptr5 | 50 | 60 | 50 | 50 |
| J83/97pptr6 | 70 | 70 | 70 | 70 |

To confirm that changes in reporter gene activity reflected changes in mRNA expression in these clones, Northern analysis was performed on induced, constitutive, and repressed clones using a radio labeled DNA probe directed towards the beta-lactamase gene. All clones that had beta-lactamase enzyme inducibility tested showed beta-lactamase mRNA inducibility. All clones that showed constitutive expression of beta-lactamase showed constitutive expression of beta-lactamase mRNA. All clones that showed repressed beta-lactamase expression showed repressed beta-lactamase mRNA. The message size of the control beta-lactamase mRNA was about 800 base pairs. The sizes of some from β-lactamase clones of the RNA were shifted higher in the gel, indicating a fusion RNA had been made between the endogenous transcript and beta-lactamase . Two known genes, CDK-6 (isolated from clone J83-PTI1) and Erg-3 (isolated from clone J89-PTI4), and two unknown genes were identified, which were isolated from clones J83PI15 and J83PI2, respectively. For clone J389-PTI4, a Northern blot was performed with the Erg-3 probe made using appropriate PCR primers determined from a published sequence which hybridizes with both the fusion RNA and the wild type RNA (for the sequence of Erg-3 see Stamminger et al., Int. Immunol. 5:63-70 (1993); for PCR methodologies, see U.S. Patent Nos: 4,800,159, 4,683,195, and 4,683,202). The inducibility in wild type Jurkat cells mimicked the beta-lactamase activity in this clone.

### Example 9 Screening of a library of known pharmacologically active modulators using a T-cell activated BLEC clone

T-cell clone J32-6D4 was used to identify potential inhibitors of the T-cell receptor pathway. This clone was selected for further study because it is difficult to identify chemicals that inhibit specific T-cell receptor pathway. Thus, this clone was used to identify chemicals that inhibit this T-cell receptor pathway that is also stimulated by the PKC activator PMA.

A first screen was performed using a generic set of 480 chemicals with known properties. The chemicals in this set were known to have pharmacological activity. Approximately one percent (7/480) of these chemicals showed greater than 50% inhibition of the PHA activation of beta-lactamase expression in clone J32-6D4 when tested in duplicate at 10 µM of chemical. Cells were activated with 1 µg/ml of PHA for 18 hours in the presence of test chemicals to test for inhibitory activity. The seven chemicals that specifically inhibited clone J32-6D4 are shown in **Table 9**. Two of these chemicals specifically inhibited clone J32-6D4 and not the control C2 cell line. This assay for the specificity of inhibition included screening these 480 chemicals for inhibitory activity using clone C2, in which the M1 muscarinic receptor was linked to a NFAT beta-lactamase reporter gene readout (see Example 7). In these experiments, the inhibition measured was the inhibition of carbachol induced expression of beta-lactamase. These results, the specific inhibition of J32-6D4 cells but not C2 cells, show that the chemicals are not toxic, do not inhibit general transcription, and do not inhibit the reporter gene product.

**TABLE 9.**

| **Active chemicals identified as exhibiting inhibitory activity of PHA activation of clone J32-6D4** | | | |
|---|---|---|---|
| **Chemical (10 µM)** | **% Inhibition of PHA activation of Clone J32-6D4** | **Inhibition of Clone C2** | **Therapeutic Category of the Chemical** |
| Digoxin | 86 | + | Cardiotonic |
| Digitoxin | 77 | + | Cardiotonic |
| Gentian Violet | 73 | + | Topical anti-infective |
| Oxyphenbuta zone | 75 | - | Anti-inflammatory |
| Mechloretha mine | 51 | - | Anti-neoplastic |
| Dipyrithione | 70 | + | Anti-bacterial |
| Ouabain | 50 | + | Cardiotonic |
| Thioguanine | 50 | + | Anti-neoplastic |

### Example 10 Screening a library of structurally characterized chemicals having unknown pharmacological properties for modulating activity of the T-cell receptor pathway using a T-cell activated BLEC clone

Having demonstrated in Example 9 that clone J32-6D4 performs robustly in a chemical screen, this clone was used to screen an additional 7,500 chemicals from a proprietary chemical library at a concentration of 10 µM per chemical. This collection of chemicals, unlike the collection of chemicals used in Example 9, contains chemicals without known pharmacological activity. Seventy-seven chemicals showed at least 50% inhibition of PHA activation of beta-lactamase expression following the general procedures set forth in Example 7. These 77 chemicals were re-tested for this activity using the same procedure and 31 chemicals were confirmed to have activity. The IC50 values of the inhibition of PHA activation of beta-lactamase expression were determined for these 31 chemicals using concentrations of chemical between about 20 µM to 2 nM. IC50 values reflect the concentration of a chemical needed to inhibit the PHA activation of the clone by 50% and were determined using known methods. These 31 chemicals were also tested for their cross inhibition of cabachol induced activation of beta-lactamase expression of clone C2 as described in Example 8.

Two chemicals, designated chemical A and chemical B, exhibited an IC50 values of about 200 nM and specifically inhibited the PHA activation of beta-lactamase expression of clone J32-6D4 but not the carbachol activation of clone C2 at the concentration tested. All of the other 31 chemicals either inhibited both clone J32-6D4 and clone C2, or had IC50 values above 1 µM.

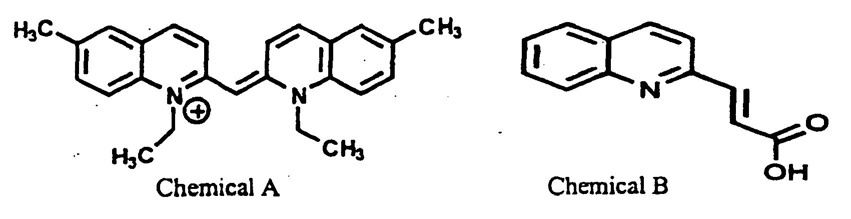

Chemicals A and B were further tested for their anti-proliferative effect on Jurkat cells and mouse L-cells (mouse fibroblast cell line). Chemical B showed no anti-proliferative effect on both the Jurkats and L-cells at concentrations up to 10 µM. Chemical A exhibited an anti-proliferative effect on the Jurkats and L-cells at 100 nM. Proliferation assays were performed by seeding about 20,000 cells unactivated by PHA into a 24 well plate. These cells were contacted with chemicals and were then incubated at 37°C for five days. The cells were contacted with 10µg/ml of MTT (Sigma Chemical Co., MO) for three hours. The cells were then collected, resuspended in isopropanol, and the absorbance was read in a plate reader at a wavelength of 570 nM with a background subtraction at a reading at a wavelength of 690 nM (see, Carmichael et al., Cancer Res. 47:936 (1987)).

### Example 11 Effects of identified chemicals on primary human T-cell proliferation.

An assay was developed to test the chemicals identified in Example 9 for their ability to inhibit the activation and proliferation of normal peripheral white blood cells to confirm their presumptive activity (see generally, Harlow and Lane, Antibodies, A Laboratory Manual, Cold Spring Harbor Press, (1988)). Peripheral blood from normal humans was drawn into heparanized Vacutainer® tubes and incubated with various concentrations of (superantigen) staphylococcal enterotoxin B (SEB, at 0.001 to 10 ng/ml) for 1 hour at 37°C. Brefeldin A, which was added and the cells were incubated an additional 5 hours. EDTA was added to detach the cells, and a 100 µL aliquot was removed, the red blood cells lysed with ammonium chloride, the remaining cells counted and their viability determined using viability staining using known methods. The red blood cells remaining in the original sample were lysed with ammonium chloride and the remaining cells (leukocytes) were permeabilized with FACS permabilizing solution using established methods. These leukocytes were harvested by centrifugation, washed and stained with the combination of antibodies CD69, IFN-γ and CD3, which were detectably labeled. Control cells consisted of cells incubated in the absence of SEB and staining control cells consisted of cells stained with CD69/MsIgG1 and CD3 antibodies, which were detectably labeled. Similar cultures will be incubated for 71 hours, pulsed with tridiated thymidine for 1 hour and harvested and the incorporated radioactivity counted by scintillation to determine a stimulation index using established methods.

Using preferred concentrations of SEB, various concentrations of cyclosporin A (CsA) were added to determine optimal conditions of CsA for blocking of SEB stimulation of peripheral blood T-cells for use as a control for non-proliferative T-cells. Controls consisted of cells incubated with culture media in place of CsA. Control cultures incubated for 1 hour were blocked with Brefeldin A for an additional 5 hours, harvested, and stained for intracellular IFN-γ or cultured for an additional 71 hours, pulsed with tritiated thymidine for one hour, harvested, and counted by liquid scintillation.

Using preferred concentrations of SEB and CsA, blood from normal donors was stimulated in the presence and absence of CsA. This established expected normal ranges for the degree of activation (% IFN-γ + activated CD3+ cells for 6 hours), proliferation (³H-TdR uptake at 72 hours) and CsA blocking at both time points.

Using preferred conditions, human blood was incubated with Chemical A or Chemical B at 2, 20, and 200 nM. CsA was used as a positive control for T-cell suppression. One hour cultures were blocked with Brefeldin A for an additional 5 hours, harvested and counted by liquid scintillation. Cell counts and percent viability were reported for each culture condition.

The results of these studies should demonstrate that at least one of the chemicals identified by the methods of the present invention have the predicted pharmacological activity in human cells.

### Example 12: Identification of genes expressed during developmental programs.

Another use of this method is for the identification of genes expressed during various cellular processes, such as developmental biology and apoptosis. Genes involved in specific developmental programs, such as the differentiation of pre-adiposites to mature adiposites, can be identified using this method.

In order to practice this method, a clone library from a pre-adiposite cell line such as 3T3-L1 is made using the methods generally described in Examples 10 to 12 above. Of course, pre-adiposite cells are used rather than Jurkat cells. This cell line can be reversible differentiated to mature adiposites by exposing them to dexamethasone and indomethasone (see, Hunt et al. Proc. Natl. Acad. Sci. U.S.A. 83:3786-3789 (1986)). These mature adiposites can be reversibly differentiated to pre-adiposites with Tumor Necrosis Factor alpha TNFa (see, Torti et al. J. Cell. Biol. 108:1105-1113 (1989)). Thus, a cell library capable of signaling the expression of genes involved in cellular differentiation can be made.

The 3T3-L1 gene trap library is FACS sorted to remove blue constitutively expressing beta-lactamase cells. The remaining green cells are then differentiated into mature adiposites using the dexamethasone and indomethasone. Blue (beta-lactamase expressing) cells are isolated using FACS. These clones represent cells in which the trapping construct integrates into a gene that is expressed in differentiated adiposites, but not in undifferentiated adiposites. This process can be repeated multiple times to insure enrichment for cells that express adiposite specific genes.

Alternatively, cell clones can be isolated which are differentiated for a specific time interval. For instance, blue and green cells differentiated for 2 days with dexamethasone and indomethasone are sorted. These populations of cells represent cells in which the trapping construct integrates into a gene that is expressed early in the differentiation process. This allows the identification of genes that are expressed during the developmental program but are not expressed in pre-adiposites or mature adiposites. This method can be used to isolated genes expressed during a variety of developmental programs, including but not limited to neuronal, cardiac, muscle, and cancer cells.

These cells lines can be used to identify genes involved in the differentiation process, and can also be used to screen chemicals that modulate the differentiation process using the methods described in Examples 8 to 10 above. Drugs that can be identified include those that enhance the growth of cells, such as neuronal cells, or depress the growth or reverse differentiation of cells, such as cancer cells.

### Example 13: Assays for modulators of G-protein coupled receptors

The general procedures of Examples 8 to 10 can be used in an analogous manner to identify cell lines suitable for screens for G-protein coupled receptors (GPCRs). GPCRs are known to signal via one of several intracellular pathways. These pathways can be activated pharmacologically in cell libraries to yield potential screening cell lines. For example, Gq coupled GPCRs are known to raise intracellular free calcium via activation of phospholipase Cb (PLCb). By isolating cell lines responsive to an increase in calcium from the genomic library (e.g. induced by ionomycin or thapsigargin), screen cell lines are generated.

For example, a calcium-sensitive clone was transfected with a Gq-type GPCR by electroporation. Cells from clone J389PTI4 were transfected by electroporation with a plasmid (pcDNA3 (Invitrogen) or pcDNA3-M1 (pcDNA3 that can operably express M1 receptor) to make cell lines J389PTI4/pcDNA3 and J389PTI4/pcDNA3-M1). Cell line J389PTI4/pcDNA3-M1 expressed the M1 receptor, whereas the cell line J389PTI4/pcDNA3 did not. Thus, the J389PTI4/pcDNA3 cell is a control cell. Two days after transfection, cells were stimulated with 20 µM carbachol in 96-well microtiter plate for 6 hours in 37 °C. These cells were contacted with CCF-2 dye for another 90 minutes. The 460/530 ratio changes were measured in a Cytoflour (Series 4000 Model) (Perceptive Biosystems) fluorescence plate reader and correspond to reporter gene expression. These results are summarized in **Table 10**. The ability of the transiently-transfected clone to detect a ligand for the GPCR demonstrates the potential of generating screening cell lines using clones made following the procedures of the present invention. The stimulation by carbachol detected in the transient tranfection assay represents a response in about 20% of the cells. To develop a stable screening cell line for the M1 receptor, this population can be sorted for individual clones responsive to carbachol and those clones can be expanded and screened to identify the most responsive clones.

Similar methods can be used to generate cell lines for Gs or Gi-coupled receptors. In these cases, clones responsive to increases or decreases in cAMP can be isolated. A variety of cell lines can be used for these procedures, such as CHO, HEK293, Neuroblastoma, P19, F11, and NT-2 cells.

**TABLE 10**

| **Cell lines that report modulation of the M1 receptor pathway** | | | |
|---|---|---|---|
| | **Relative expression of beta-lactamase in cells Exposed to the indicated stimuli** | | |
| **Cell Line** | **Unstimulated** | **30** µ**M Carbachol** | **10 nM PHA** |
| J389PTI4/pcDNA3 | 1 | 1 | 12 |
| J389PTI4/pcDNA3-M1 | 1 | 4 | 13 |

### Publications

### Articles

G. Friedrich, P. Soriano, Methods in Enzymology, Vol. 225: 681 (1993)
G. Friedrich, P. Soriano, Genes & Development, Vol. 5: 1513 (1991)
A. Gossler, et al., Reports, 28 April: 463 (1989)
D. Hill, W. Wurst, Methods in Enzymology, Vol. 225: 664 (1993)
P. Mountford, A. Smith, TIG, Vol. 11 No. 5: 179 (1995)
P. Mountford, et al., Proc. Natl. Acad. Sci, USA, Vol. 91: 4303 (1994)
Q. Niwa, et al., J. Biochem, Vol. 13: 343 (1993)
R. Reddy, et al., Proc. Natl. Acad. Sci. USA, Vol. 89: 6721 (1992)
S. Shapiro, P. Senapathy, Nucleic Acids Research, Vol. 17, No. 17: 7155 (1987)
T. Skarnes, et al., Genes & Development, Vol. 6: 903 (1992)
W. Wurst, et al., Genetics, Vol. 139: 889 (1995)

## Claims

1. A method for determining a cellular response profile for a target, comprising:
i) introducing a target into a first plurality of eukaryotic cells, wherein each eukaryotic cell comprises a genomic polynucleotide operably linked to a promoterless β-lactamase polynucleotide encoding a β-lactamase,
ii) inducing expression of said target in said first plurality of eukaryotic cells or contacting said first plurality of eukaryotic cells with a ligand, inhibitor or activator of said target,
iii) separating by FACS a second plurality of cells exhibiting an increase or decrease in expression of said β-lactamase in response to step (ii),
iv) identifying a plurality of genomic polynucleotides exhibiting an increase or decrease in expression present in said second plurality of cells, wherein an increase or decrease in expression of each said genomic polynucleotides from said plurality of polynucleotides provides a profile of cellular response relating to said target,
wherein said promoterless β-lactamase polynucleotide was introduced into said first plurality of eukaryotic cells via a viral vector.

2. A method for determining a cellular response profile for a chemical, comprising:
i) contacting a first plurality of eukaryotic cells, wherein each eukaryotic cell comprises a genomic polynucleotide operably linked to a promoterless β-lactamase polynucleotide, with said chemical,
ii) separating by FACS a second plurality of cells exhibiting an increase or decrease in expression of β-lactamase in response to contact with said chemical,
iii) identifying a plurality of genomic polynucleotides exhibiting an increase or decrease in expression present in said second plurality of cells,
wherein an increase or decrease in expression of each said genomic polynucleotides from said plurality of polynucleotides provides a profile of cellular response relating to said chemical, wherein said promoterless β-lactamase polynucleotide was introduced into said first plurality of eukaryotic cells via a viral vector

3. A method for screening compounds for activity as an activator or inhibitor of a target, comprising:
i) introducing a target into a first plurality of eukaryotic cells, wherein each eukaryotic cell comprises a genomic polynucleotide operably linked to a promoterless polynucleotide encoding β-lactamase,
ii) inducing expression of said target in said first plurality of eukaryotic cells or contacting said first plurality of eukaryotic cells with a ligand, inhibitor or activator of said target,
iii) separating by FACS a second plurality of cells exhibiting an increase or decrease in expression of β-lactamase in response to step (ii),
iv) contacting a cell derived from said second plurality of cells with a test chemical,
v) optionally inducing expression of said target in said cell or contacting said cell with a ligand, inhibitor or activator of said target,
vi) determining whether said test chemical increases or decreases β-lactamase activity compared to a control cell which was not contacted with said test chemical,
wherein said promoterless β-lactamase polynucleotide was introduced into said first plurality of eukaryotic cells via a viral vector.

4. A method for developing a cell sensor panel, comprising:
i) introducing a target into a first plurality of eukaryotic cells, wherein each eukaryotic cell comprises a genomic polynucleotide operably linked to a promoterless β- lactamase polynucleotide encoding a β-lactamase,
ii) inducing expression of said target in said first plurality of eukaryotic cells or contacting said first plurality of eukaryotic cells with a ligand, inhibitor or activator of said target,
iii) separating by FACS a second plurality of cells exhibiting an increase or decrease in expression of said β-lactamase in response to step (ii),
iv) selecting cells for said cell sensor panel from said second plurality of cells exhibiting a 1.5 to 40 fold change in the 460/530 ratio of the measured fluorescence in response to the induction of expression of said target in said clonal cells, or in response to exposure of said clonal cells to a ligand, inhibitor or activator for said target, and
wherein said promoterless β-lactamase polynucleotide was introduced into said first plurality of eukaryotic cells via a viral vector.

5. The method of any of claims 1-4, wherein said viral vector comprises a nucleic acid molecule comprising a splice acceptor sequence, a splice donor sequence,
wherein said splice acceptor sequence flanks said polynucleotide encoding said β-lactamase, and said splice donor sequence flanks said polynucleotide encoding said β-lactamase.

6. The method of claim 5, wherein said viral vector further comprises a translational start sequence for said β-lactamase.

7. The method of claim 1, wherein said second plurality of cells exhibits a 1.5 to 40 fold change in the 460/530 ratio of the measured fluorescence in response to the induction of expression of said target in said first plurality of eukaryotic cells or contacting said first plurality of eukaryotic cells with a ligand, inhibitor or activator of said target.

8. The method of claim 6, wherein said translational start sequence comprises a Kozak sequence.

9. The method of claim 5, wherein said viral vector further comprises an internal ribosome entry site.

10. The method of claim 5, wherein said viral vector further comprises a poly-adenylation site for said β-lactamase.

11. The method of claim 5, wherein said viral vector is a retrovirus.

12. The method of claim 5, wherein said first plurality of eukaryotic cells comprises at least 10,000 cell populations.

13. The method of claim 5, wherein said first plurality of eukaryotic cells comprises at least 100,000 cell populations.

14. A cell sensor panel, comprising,
a plurality of clonal cells, wherein each clonal cell comprises a distinct genomic polynucleotide operably linked to a promoterless β- lactamase polynucleotide encoding a β-lactamase, and
wherein said clonal cells exhibit a 1.5 to 40 fold change in the 460/530 ratio of the measured fluorescence in response to the induction of expression of said target in said clonal cells, or in response to exposure of said clonal cells to a ligand, inhibitor or activator for said target, and
wherein said promoterless β-lactamase polynucleotide was introduced into said clonal cells via a viral vector provided that said clonal cells are not obtained by a process comprising the step of using human embryos for generating human embryonic stem cells.

15. A cell sensor panel, comprising,
a plurality of clonal cells, wherein each said clonal cell comprises a distinct genomic polynucleotide operably linked with a promoterless β-lactamase expression construct, and
wherein said clonal cells exhibit a 1.5 to 40 fold change in the 460/530 ratio of the measured fluorescence in response to contact of a test chemical with said clonal cells, and
wherein said clonal cells were selected from a population of cells into which a viral vector has been introduced, and wherein said viral vector lacks a promoter to express said β-lactamase provided that said clonal cells are not obtained by a process comprising the step of using human embryos for generating human embryonic stem cells.

16. The cell sensor panel of claim 14 or 15, wherein said panel further comprises at least one cell line wherein said β-lactamase expression is under the control of response element.

17. The cell sensor panel of claim 14 or 15, wherein said panel is in a multiwell plate.

18. The cell sensor panel of claim 14 or 15, wherein said clonal cells are derived from embryonic or hematopoetic stem cells, provided that the embryonic stem cells are not obtained by a process comprising the step of using human embryos for generating human embryonic stem cells.

## Patentansprüche

1. Verfahren zur Bestimmung eines zellulären Reaktionsprofils für ein Ziel, umfassend:
i) Einbringen eines Ziels in eine erste Vielzahl von eukaryontischen Zellen, wobei jede eukaryontische Zelle ein genomisches Polynukleotid umfasst, das operabel mit einem promotorlosen β-Lactamase-Polynukleotid, das eine β-Lactamase kodiert, verbunden ist,
ii) Induzieren der Expression des Ziels in der ersten Vielzahl von eukaryontischen Zellen oder In-Kontakt-Bringen der ersten Vielzahl von eukaryontischen Zellen mit einem Liganden, Inhibitor oder Aktivator des Ziels,
iii) Abtrennen durch FACS einer zweiten Vielzahl von Zellen, die eine Zunahme oder Abnahme der Expression der β-Lactamase in Reaktion auf Schritt (ii) aufweisen,
iv) Identifizieren einer Vielzahl von genomischen Polynukleotiden, die eine Zunahme oder Abnahme der Expression in der zweiten Vielzahl von Zellen aufweisen, wobei eine Zunahme oder Abnahme der Expression eines jeden der genomischen Polynukleotide aus der Vielzahl von Polynukleotiden ein Profil einer zellulären Reaktion, das mit dem Ziel in Verbindung steht, bereitstellt,
wobei das promotorlose β-Lactamase-Polynukleotid in die erste Vielzahl von eukaryontischen Zellen mittels eines viralen Vektors eingebracht wurde.

2. Verfahren zur Bestimmung eines zellulären Reaktionsprofils für eine Chemikalie, umfassend:
i) In-Kontakt-Bringen einer ersten Vielzahl von eukaryontischen Zellen, wobei jede eukaryontische Zelle ein genomisches Polynukleotid umfasst, das operabel mit einem promotorlosen β-Lactamase-Polynukleotid verbunden ist, mit der Chemikalie,
ii) Abtrennen durch FACS einer zweiten Vielzahl von Zellen, die eine Zunahme oder Abnahme der Expression von β-Lactamase in Reaktion auf ein In-Kontakt-Bringen mit der Chemikalie aufweisen,
iii) Identifizieren einer Vielzahl von genomischen Polynukleotiden, die eine Zunahme oder Abnahme der Expression in der zweiten Vielzahl von Zellen aufweisen, wobei eine Zunahme oder Abnahme der Expression eines jeden der genomischen Polynukleotide aus der Vielzahl von Polynukleotiden ein Profil einer zellulären Reaktion, das mit der Chemikalie in Verbindung steht, bereitstellt,
wobei das promotorlose β-Lactamase-Polynukleotid in die erste Vielzahl von eukaryontischen Zellen mittels eines viralen Vektors eingebracht wurde.

3. Verfahren zum Screenen von Verbindungen bezüglich einer Aktivität als Aktivator oder Inhibitor eines Ziels, umfassend:
i) Einbringen eines Ziels in eine erste Vielzahl von eukaryontischen Zellen, wobei jede eukaryontische Zelle ein genomisches Polynukleotid umfasst, das operabel mit einem promotorlosen, für β-Lactamase kodierenden Polynukleotid verbunden ist,
ii) Induzieren der Expression des Ziels in der ersten Vielzahl von eukaryontischen Zellen oder In-Kontakt-Bringen der ersten Vielzahl von eukaryontischen Zellen mit einem Liganden, Inhibitor oder Aktivator des Ziels,
iii) Abtrennen durch FACS einer zweiten Vielzahl von Zellen, die eine Zunahme oder Abnahme der Expression von β-Lactamase in Reaktion auf Schritt (ii) aufweisen,
iv) In-Kontakt-Bringen einer Zelle, die von der zweiten Vielzahl von Zellen abgeleitet ist, mit einer Test-Chemikalie,
v) gegebenenfalls Induzieren der Expression des Ziels in der Zelle oder In-Kontakt-Bringen der Zelle mit einem Liganden, Inhibitor oder Aktivator des Ziels,
vi) Bestimmen, ob die Test-Chemikalie β-Lactamase-Aktivität im Vergleich zu einer Kontrollzelle, die nicht mit der Test-Chemikalie in Kontakt gebracht wurde, erhöht oder verringert,
wobei das promotorlose β-Lactamase-Polynukleotid in die erste Vielzahl von eukaryontischen Zellen mittels eines viralen Vektors eingebracht wurde.

4. Verfahren zur Entwicklung eines Zellsensorelements, umfassend:
i) Einbringen eines Ziels in eine erste Vielzahl von eukaryontischen Zellen, wobei jede eukaryontische Zelle ein genomisches Polynukleotid umfasst, das operabel mit einem promotorlosen β-Lactamase-Polynukleotid, das eine β-Lactamase kodiert, verbunden ist,
ii) Induzieren der Expression des Ziels in der ersten Vielzahl von eukaryontischen Zellen oder In-Kontakt-Bringen der ersten Vielzahl von eukaryontischen Zellen mit einem Liganden, Inhibitor oder Aktivator des Ziels,
iii) Abtrennen durch FACS einer zweiten Vielzahl von Zellen, die eine Zunahme oder Abnahme der Expression der β-Lactamase in Reaktion auf Schritt (ii) aufweisen,
iv) Auswählen von Zellen für das Zellsensorelement aus der zweiten Vielzahl von Zellen, die eine 1,5- bis 40-fache Änderung des 460/530-Verhältnisses der gemessenen Fluoreszenz in Reaktion auf die Induktion einer Expression des Ziels in den klonalen Zellen oder in Reaktion auf eine Exposition der klonalen Zellen gegenüber einem Liganden, Inhibitor oder Aktivator für das Ziel aufweisen,
wobei das promotorlose β-Lactamase-Polynukleotid in die erste Vielzahl von eukaryontischen Zellen mittels eines viralen Vektors eingebracht wurde.

5. Verfahren nach einem der Ansprüche 1-4, wobei der virale Vektor ein Nukleinsäuremolekül umfasst, das eine Splice-Akzeptor-Sequenz und eine Splice-Donor-Sequenz umfasst, wobei die Splice-Akzeptor-Sequenz das Polynukleotid, das die β-Lactamase kodiert, flankiert und die Splice-Donor-Sequenz das Polynukleotid, das die β-Lactamase kodiert, flankiert.

6. Verfahren nach Anspruch 5, wobei der virale Vektor ferner eine Translationsstartsequenz für die β-Lactamase umfasst.

7. Verfahren nach Anspruch 1, wobei die zweite Vielzahl von Zellen eine 1,5-bis 40-fache Änderung des 460/530-Verhältnisses der gemessenen Fluoreszenz in Reaktion auf die Induktion der Expression des Ziels in der ersten Vielzahl von eukaryontischen Zellen oder das In-Kontakt-Bringen der ersten Vielzahl von eukaryontischen Zellen mit einem Ligand, Inhibitor oder Aktivator des Ziels aufweist.

8. Verfahren nach Anspruch 6, wobei die Translationsstartsequenz eine Kozak-Sequenz umfasst.

9. Verfahren nach Anspruch 5, wobei der virale Vektor ferner eine innen liegende Ribosomeneintrittsstelle umfasst.

10. Verfahren nach Anspruch 5, wobei der virale Vektor ferner eine Polyadenylierungsstelle für die β-Lactamase umfasst.

11. Verfahren nach Anspruch 5, wobei der virale Vektor ein Retrovirus ist.

12. Verfahren nach Anspruch 5, wobei die erste Vielzahl von eukaryontischen Zellen mindestens 10000 Zellpopulationen umfasst.

13. Verfahren nach Anspruch 5, wobei die erste Vielzahl von eukaryontischen Zellen mindestens 100000 Zellpopulationen umfasst.

14. Zellsensorelement, das eine Vielzahl von klonalen Zellen umfasst, wobei jede klonale Zelle ein anderes genomisches Polynukleotid umfasst, das operabel mit einem promotorlosen β-Lactamase-Polynukleotid, das eine β-Lactamase kodiert, verbunden ist, wobei die klonalen Zellen eine 1,5- bis 40-fache Änderung des 460/530-Verhältnisses der gemessenen Fluoreszenz in Reaktion auf die Induktion der Expression des Ziels in den klonalen Zellen oder in Reaktion auf eine Exposition der klonalen Zellen gegenüber einem Ligand, Inhibitor oder Aktivator für das Ziel aufweisen und das promotorlose β-Lactamase-Polynukleotid in die klonalen Zellen mittels eines viralen Vektors eingebracht wurde, mit der Maßgabe, dass die klonalen Zellen nicht durch ein Verfahren erhalten werden, das den Schritt eines Verwendens von menschlichen Embryonen für eine Erzeugung menschlicher embryonaler Stammzellen umfasst.

15. Zellsensorelement, das eine Vielzahl von klonalen Zellen umfasst, wobei eine jede der klonalen Zellen ein anderes genomisches Polynukleotid umfasst, das operabel mit einem promotorlosen β-Lactamase-Expressionskonstrukt verbunden ist, wobei die klonalen Zellen eine 1,5- bis 40-fache Änderung des 460/530-Verhältnisses der gemessenen Fluoreszenz in Reaktion auf ein In-Kontakt-Bringen einer Test-Chemikalie mit den klonalen Zellen aufweisen, die klonalen Zellen aus einer Population von Zellen ausgewählt wurden, in die ein viraler Vektor eingebracht wurde, und der virale Vektor keinen Promotor für eine Expression der β-Lactamase aufweist, mit der Maßgabe, dass die klonalen Zellen nicht durch ein Verfahren erhalten werden, das den Schritt einer Verwendung von menschlichen Embryonen für eine Erzeugung menschliche embryonaler Stammzellen umfasst.

16. Zellsensorelement nach Anspruch 14 oder 15, wobei das Element ferner mindestens eine Zelllinie umfasst, wobei die β-Lactamase-Expression unter der Kontrolle eines Reaktionselements steht.

17. Zellsensorelement nach Anspruch 14 oder 15, wobei sich das Element in einer Multiwell-Platte befindet.

18. Zellsensorelement nach Anspruch 14 oder 15, wobei die klonalen Zellen von embryonalen oder hämatopoetischen Stammzellen abgeleitet sind, mit der Maßgabe, dass die embryonalen Stammzellen nicht durch ein Verfahren erhalten werden, das den Schritt eines Verwendens von menschlichen Embryonen für eine Erzeugung menschlicher embryonaler Stammzellen umfasst.

## Revendications

1. Procédé pour la détermination d'un profil de réponse cellulaire pour une cible, comprenant :
i) l'introduction d'une cible dans une première pluralité de cellules eucaryotes, tandis que chaque cellule eucaryote comprend un polynucléotide génomique lié de manière opérationnelle à un polynucléotide de β-lactamase exempt de promoteur, codant pour une β-lactamase,
ii) l'induction de l'expression de ladite cible dans ladite première pluralité de cellules eucaryotes ou la mise en contact de ladite première pluralité de cellules eucaryotes avec un ligand, un inhibiteur ou un activateur de ladite cible,
iii) la séparation par FACS d'une deuxième pluralité de cellules présentant une augmentation ou une diminution de l'expression de ladite β-lactamase en réponse à l'étape ii),
iv) l'identification d'une pluralité de polynucléotides génomiques présentant une augmentation ou une diminution de l'expression présents dans ladite deuxième pluralité de cellules, tandis qu'une augmentation ou une diminution de l'expression de chacun des dits polynucléotides génomiques de ladite pluralité de polynucléotides procure un profil de réponse cellulaire concernant ladite cible,
tandis que ledit polynucléotide de β-lactamase exempt de promoteur a été introduit dans ladite première pluralité de cellules eucaryotes via un vecteur viral.

2. Procédé pour la détermination d'un profil de réponse cellulaire pour un produit chimique, comprenant :
i) la mise en contact d'une première pluralité de cellules eucaryotes, tandis que chaque cellule eucaryote comprend un polynucléotide génomique lié de manière opérationnelle à un polynucléotide de β-lactamase exempt de promoteur, avec ledit produit chimique,
ii) la séparation par FACS d'une deuxième pluralité de cellules présentant une augmentation ou une diminution de l'expression de β-lactamase en réponse à la mise en contact avec ledit produit chimique,
iii) l'identification d'une pluralité de polynucléotides génomiques présentant une augmentation ou une diminution de l'expression présents dans ladite deuxième pluralité de cellules, tandis qu'une augmentation ou une diminution de l'expression de chacun des dits polynucléotides génomiques de ladite pluralité de polynucléotides procure un profil de réponse cellulaire concernant ledit produit chimique,
tandis que ledit polynucléotide de β-lactamase exempt de promoteur a été introduit dans ladite première pluralité de cellules eucaryotes via un vecteur viral.

3. Procédé pour le criblage de composés pour leur activité comme activateur ou inhibiteur d'une cible, comprenant :
i) l'introduction d'une cible dans une première pluralité de cellules eucaryotes, tandis que chaque cellule eucaryote comprend un polynucléotide génomique lié de manière opérationnelle à un polynucléotide exempt de promoteur, codant pour la β-lactamase,
ii) l'induction de l'expression de ladite cible dans ladite première pluralité de cellules eucaryotes ou la mise en contact de ladite première pluralité de cellules eucaryotes avec un ligand, un inhibiteur ou un activateur de ladite cible,
iii) la séparation par FACS d'une deuxième pluralité de cellules présentant une augmentation ou une diminution de l'expression de β-lactamase en réponse à l'étape ii),
iv) la mise en contact d'une cellule dérivée de ladite deuxième pluralité de cellules avec un composé chimique pour test,
v) en option l'induction de l'expression de ladite cible dans ladite cellule ou la mise en contact de ladite cellule avec un ligand, un inhibiteur ou un activateur de ladite cible,
vi) la détermination de ce que ledit produit chimique pour test augmente ou diminue ou non l'activité de β-lactamase par comparaison avec une cellule témoin qui n'a pas été mise en contact avec ledit composé chimique pour test,
tandis que ledit polynucléotide de β-lactamase exempt de promoteur a été introduit dans ladite première pluralité de cellules eucaryotes via un vecteur viral.

4. Procédé pour le développement d'un panel de détecteurs cellulaires, comprenant :
i) l'introduction d'une cible dans une première pluralité de cellules eucaryotes, tandis que chaque cellule eucaryote comprend un polynucléotide génomique lié de manière opérationnelle à un polynucléotide de β-lactamase exempt de promoteur, codant pour une β-lactamase,
ii) l'induction de l'expression de ladite cible dans ladite première pluralité de cellules eucaryotes ou la mise en contact de ladite première pluralité de cellules eucaryotes avec un ligand, un inhibiteur ou un activateur de ladite cible,
iii) la séparation par FACS d'une deuxième pluralité de cellules présentant une augmentation ou une diminution de l'expression de ladite β-lactamase en réponse à l'étape ii),
iv) la sélection de cellules pour ledit panel de détecteurs cellulaires à partir de ladite deuxième pluralité de cellules présentant une modification de 1,5 à 40 fois dans le rapport 460/530 de la fluorescence mesurée en réponse à l'induction de l'expression de ladite cible dans les dites cellules clonales, ou en réponse à une exposition des dites cellules clonales à un ligand, un inhibiteur ou un activateur pour ladite cible, et
tandis que ledit polynucléotide de β-lactamase exempt de promoteur a été introduit dans ladite première pluralité de cellules eucaryotes via un vecteur viral.

5. Procédé selon l'une quelconque des revendications 1-4, dans lequel ledit vecteur viral comprend une molécule d'acide nucléique comportant une séquence d'accepteur d'épissage, une séquence de donneur d'épissage,
tandis que ladite séquence d'accepteur d'épissage encadre ledit polynucléotide codant pour ladite β-lactamase, et ladite séquence de donneur d'épissage encadre ledit polynucléotide codant pour ladite β-lactamase.

6. Procédé selon la revendication 5, dans lequel ledit vecteur viral comprend en outre une séquence d'initiation traductionnelle pour ladite β-lactamase.

7. Procédé selon la revendication 1, dans lequel ladite deuxième pluralité de cellules présente une modification de 1,5 à 40 fois dans le rapport 460/530 de la fluorescence mesurée en réponse à l'induction de l'expression de ladite cible dans ladite première pluralité de cellules eucaryotes ou la mise en contact de ladite première pluralité de cellules eucaryotes avec un ligand, un inhibiteur ou un activateur de ladite cible.

8. Procédé selon la revendication 6, dans lequel ladite séquence d'initiation traductionnelle comprend une séquence de Kozak.

9. Procédé selon la revendication 5, dans lequel ledit vecteur viral comprend en outre un site d'entrée de ribosome interne.

10. Procédé selon la revendication 5, dans lequel ledit vecteur viral comprend en outre un site de poly-adénylation pour ladite β-lactamase.

11. Procédé selon la revendication 5, dans lequel ledit vecteur viral est un rétrovirus.

12. Procédé selon la revendication 5, dans lequel ladite première pluralité de cellules eucaryotes comprend des populations d'au moins 10.000 cellules.

13. Procédé selon la revendication 5, dans lequel ladite première pluralité de cellules eucaryotes comprend des populations d'au moins 100.000 cellules.

14. Panel de détecteurs cellulaires, comprenant :
une pluralité de cellules clonales, tandis que chaque cellule clonale comprend un polynucléotide génomique distinct lié de manière opérationnelle à un polynucléotide de β-lactamase exempt de promoteur, codant pour une β-lactamase, et
tandis que les dites cellules clonales présentent une modification de 1,5 à 40 fois dans le rapport 460/530 de la fluorescence mesurée en réponse à l'induction de l'expression de ladite cible dans les dites cellules clonales, ou en réponse à l'exposition des dites cellules clonales à un ligand, un inhibiteur ou un activateur pour ladite cible, et
tandis que ledit polynucléotide de β-lactamase exempt de promoteur a été introduit dans les dites cellules clonales via un vecteur viral, à condition que les dites cellules clonales ne soient pas obtenues par un procédé comprenant l'étape d'utilisation d'embryons humains pour l'obtention de cellules souches embryonnaires humaines.

15. Panel de détecteurs cellulaires, comprenant :
une pluralité de cellules clonales, tandis que chacune des dites cellules clonales comprend un polynucléotide génomique distinct lié de manière opérationnelle à une construction d'expression de β-lactamase exempte de promoteur, et
tandis que les dites cellules clonales présentent une modification de 1,5 à 40 fois dans le rapport 460/530 de la fluorescence mesurée en réponse à un contact d'un produit chimique pour test avec lesdites cellules clonales, et
tandis que les dites cellules clonales ont été sélectionnées à partir d'une population de cellules dans lesquelles un vecteur viral a été introduit, et tandis que ledit vecteur viral est dénué de promoteur pour l'expression de ladite β-lactamase, à condition que les dites cellules clonales ne soient pas obtenues par un procédé comprenant l'étape d'utilisation d'embryons humains pour l'obtention de cellules souches embryonnaires humaines.

16. Panel de détecteurs cellulaires selon la revendication 14 ou 15, dans lequel ledit panel comprend en outre au moins une lignée cellulaire dans laquelle ladite expression de β-lactamase est sous le contrôle d'un élément de réponse.

17. Panel de détecteurs cellulaires selon la revendication 14 ou 15, dans lequel ledit panel est une plaque multi-puits.

18. Panel de détecteurs cellulaires selon la revendication 14 ou 15, dans lequel les dites cellules clonales sont dérivées de cellules souches embryonnaires ou hématopoïétiques, à condition que les cellules souches embryonnaires ne soient pas obtenues par un procédé comprenant l'étape d'utilisation d'embryons humains pour l'obtention de cellules souches embryonnaires humaines.
